(19) Europäisches Patentamt
European Patent Office
Office européen des brevets

(11) EP 1 957 665 B1

(12) **EUROPEAN PATENT SPECIFICATION**

(45) Date of publication and mention of the grant of the patent:
**05.05.2010 Bulletin 2010/18**

(21) Application number: **06831651.2**

(22) Date of filing: **06.12.2006**

(51) Int Cl.:
***C12Q 1/18*** *(2006.01)*

(86) International application number:
**PCT/IB2006/003489**

(87) International publication number:
**WO 2007/066206 (14.06.2007 Gazette 2007/24)**

(54) **METHOD FOR IDENTIFYING AND/OR QUANTIFYING ANTI-INFECTIVE COMPOUNDS**

VERFAHREN ZUR IDENTIFIZIERUNG UND/ODER QUANTIFIZIERUNG ANTIINFEKTIÖSER VERBINDUNGEN

PROCEDE D'IDENTIFICATION ET/OU DE QUANTIFICATION DE COMPOSES ANTI-INFECTIEUX

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.12.2005 US 742554 P**

(43) Date of publication of application:
**20.08.2008 Bulletin 2008/34**

(73) Proprietor: **Merlion Pharmaceuticals SA**
**1201 Genève (CH)**

(72) Inventors:
- **BENGHEZAL, Mohammed**
  **Nedlands 6009 WA Perth (AU)**
- **ADAM, Eric**
  **FIND**
  **1216 Cointrin/Geneva (FR)**
- **BRAILLARD, Stephanie**
  **CH-1026 Denges (CH)**
- **BURN, Christine**
  **08006 Barcelona (ES)**
- **LUCAS, Aurore**
  **F-74970 Marignier (FR)**
- **PACCAUD, Jean-Pierre**
  **CH-1233 Sezenove (CH)**
- **VALENTINO, Emilio**
  **CH-1030 Busigny (CH)**
- **COSSON, Pierre**
  **CH-1227 Carouge (CH)**

(74) Representative: **Webster, Jeremy Mark et al**
**Mewburn Ellis LLP**
**33 Gutter Lane**
**London**
**EC2V 8AS (GB)**

(56) References cited:
**WO-A2-02/101081**

EP 1 957 665 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### The field of the invention

The present invention relates to a method for identifying and/or quantifying the anti-infective activity of a compound. Furthermore it relates to a method for identifying and/or quantifying a mutation in a virulence factor of a pathogen of a host organism that reduces the virulence of the pathogen to a unicellular test organism.

### Background of the invention

The infectivity of a pathogen and the pathogenesis involves the interaction of a pathogen with a host cell. It has been shown, that factors which affect pathogenesis in plants or animals can also affect pathogenesis in lower eukaryotic organisms. For example, the same bacterial virulence factors of Pseudomonas aeruginosa (P. aeruginosa), which affect the worm Caenorhabditis elegans (C. elegans), also affect Arabidopsis thaliana and show virulence in a mammalian mouse assay as described in WO 98/50080. However, approaches for testing infectivity and in particular virulence using multicellular organisms are timeconsuming and expensive.

A method using a unicellular organism for the assessment of the virulence of a pathogen has been described in WO 02/101081. The unicellular amoeba Dictyostelium discoideum DH1-10 has been used to assess the virulence of a pathogen to a host organism by exposing a culture of Dictyostelium discoideum DH1-10 to the pathogen and monitoring the growth of Dictyostelium in the presence of the pathogen. The method described in WO 02/101081 has some limits with regard to sensitivity, reproducibility and quantification of the virulence of a pathogen, however. Since the fitness of a cultured organism depends on the culture conditions which might vary due to slight changes in the medium or the temperature of the culture, the sensitivity of the test unicellular organism with regard to the pathogen might change and therefore it will be difficult to quantify and reproduce the respective virulence test. Furthermore, the assessment of the virulence of a pathogen with the method desribed in WO 02/101081 is only semiquantitative since growth of Dictyostelium is monitored visually by measuring the colony diameter or by measuring the cytoskeleton of the organism with a light microscope. Due to the fact that the growth of the test unicellular organism is monitored, the assay described in WO 02/101081 is very time consuming i.e. the test unicellular organism has to be exposed to the pathogen for 4-7 days. This long period of exposition might be as well disadvantageous with regard to a potential decomposition of the compound to be tested

Therefore, there is a need in the art for less time consuming, more sensitive and more reproducible unicellular test host systems for testing pathogen infectivity which can be used for measuring quantitatively the virulence of a pathogen and the anti-infective activity of a compound.

### Summary of the invention

The object of the present invention is to provide improved methods for testing pathogen infectivity which does not have the above-mentioned drawbacks. In a first aspect, the present invention provides a method for identifying and/or quantifying the anti-infective activity of an anti-infective compound, said method comprising:

(a) exposing an unicellular test organism to a pathogen of a host organism in the absence of the anti-infective compound to be identified and/or quantified;
(b) determining the ratio of the concentration of the unicellular test organism to that of the pathogen which allows the pathogen to grow in the presence of the unicellular test organism;
(c) exposing the unicellular test organism to the pathogen at the ratio determined in step (b) in the presence of the anti-infective compound to be identified and/or quantified;
(d) exposing the unicellular test organism to the pathogen at the ratio determined in step (b) in the absence of the anti-infective compound to be identified and/or quantified; and
(e) monitoring the growth of the pathogen in the presence and in the absence of the anti-infective compound to be identified and/or quantified, wherein a lower level of growth of the pathogen in the presence of the anti-infective compound to be identified and/or quantified than observed in the absence of the anti-infective compound to be identified and/or quantified indicates that the anti-infective compound to be identified and/or quantified has an anti-infective activity.

Suitably the method is a method for quantifying $IC_{50}$ of an anti-infective compound, said method comprising:

(a) exposing an unicellular test organism to a pathogen of a host organism in the absence of the anti-infective compound;

(b) determining the ratio of the concentration of the unicellular test organism to that of the pathogen which allows the pathogen to grow in the presence of the unicellular test organism;
(c) exposing the unicellular test organism to the pathogen at the ratio determined in step (b) independently in the presence of two or more concentrations of the anti-infective compound;
(d) exposing the unicellular test organism to the pathogen at the ratio determined in step (b) in the absence of the anti-infective compound; and
(e) monitoring the growth of the pathogen independently in the presence of two or more concentrations of the anti-infective compound and in the absence of the anti-infective compound, wherein the level of growth of the pathogen in the presence of two or more concentrations of the anti-infective compound and the level of growth of the pathogen in the absence of the anti-infective compound are used to calculate the $IC_{50}$ of the anti-infective compound.

A further aspect of the present invention is a method for identifying and/or quantifying a mutation in a virulence factor of a pathogen of a host organism that reduces the virulence of the pathogen to a unicellular test organism, said method comprising:

(a) selecting a pathogen of a host, wherein the pathogen is able to grow in the presence of the unicellular test organism;
(b) selecting a mutant of the pathogen;
(c) exposing the unicellular test organism to the pathogen;
(d) determining the ratio of the concentration of the unicellular test organism to that of the pathogen which allows the pathogen to grow in the presence of the unicellular test organism;
(e) exposing the unicellular test organism to the pathogen at the ratio determined in step (b);
f) exposing the unicellular test organism to the mutant of the pathogen at the ratio determined in step (b);
g) monitoring the growth of the pathogen and the growth of the mutant of the pathogen,

wherein a higher level of growth of the pathogen than observed for the mutant of the pathogen indicates that the mutant of the pathogen has a mutation in the virulence factor of the pathogen that reduces the virulence of the pathogen to the host organism.
Other objects and advantages will become apparent to those skilled in the art from a review of the ensuing detailed description, which proceeds with reference to the following illustrative drawings, and the attendant claims.

**Brief description of the figures**

**Figure 1** shows the obtained matrix of the Klebsiella pneumoniae assay and the evaluation of the phenotype of the mutants and the potency of compound ATH18534. The rows A, J to L and N to P are wild type strain Kp52145 (BAT39). Rows B to I are mutant strains, BAT21, BAT30, BAT27, BAT 146, BAT168, BAT172, BAT187 and BAT199, respectively. The row M is the wild-type strain treated with 10 μM of compound ATH18534 (3-(allylsulfanyl)-5-hydroxy-6-methyl-1,2,4-triazine).

**Figure 2** shows OD values corresponding to the obtained matrix (Figure 1) of the Klebsiella pneumoniae assay and the evaluation of the phenotype of the mutants and the potency of compound ATH18534. The rows of the two blocks (50'000-9'250 and 6'250-0) are numbered with A-P from top to bottom as shown in figure 1. The rows A, J to L and N to P are wild type strain Kp52145 (BAT39). Rows B to I are mutant strains, BAT21, BAT30, BAT27, BAT 146, BAT168, BAT172, BAT187 and BAT189, respectively. The row M is the wild-type strain treated with 10 μM of compound ATH18534 (3-(allylsulfanyl)-5-hydroxy-6-methyl-1,2,4-triazine).

**Figure 3** shows the antibiotic (abscissa) and avirulence (ordinate) activities of the screening plates of the Klebsiella pneumoniae assay.

**Figure 4** shows OD values of the E. coli assay and the evaluation of the phenotype of the mutants and the potency of compound ATH18534. The rows of the two blocks (50'000-9'250 and 6'250-0) are numbered with A-P from top to bottom as shown in figure 1. The rows A, J to P are wild type strain BAT452. Rows B to I are mutant strains, BAT501, BAT502, BAT503, BAT 505, BAT546, BAT549, BAT550 and BAT563, respectively.

**Figure 5** shows the antibiotic (abscissa) and avirulence (ordinate) activities of the screening plates of the E. coli assay.

**Figure 6** shows OD values of the Pseudomonas aeruginosa assay and the evaluation of the phenotype of the mutants and the potency of compound ATH18534. The rows of the two blocks (50'000-9'250 and 6'250-0) are numbered with A-P from top to bottom as shown in figure 1. The rows A, J to P are wild type strain BAT10. In row J bacteria were omitted for $T_p$ concentration of 18,500 to 9,250 cells/ml). Rows B to I are mutant strains, BAT331, BAT2, BAT334, BAT357, BAT370, BAT375, BAT406 and BAT408, respectively.

**Figure 7** shows the antibiotic (abscissa) and avirulence (ordinate) activities of the screening plates of the Pseudomonas aeruginosa assay.

**Figure 8** shows $IC_{50}$ of ATH18534 (3-(allylsulfanyl)-5-hydroxy-6-methyl-1,2,4-triazine) in the Tetrahymena pyriformis-Klebsiella pneumoniae assay. (A) Micro-plate format for compound dose-response. (B) Average of quadruplicates of the activity and OD450 of ATH18534 compound. (C) Sigmoidal curves were drawn and $IC_{50}$ determined.

**Figure 9** shows the Sub-MIC effect of Trimethoprim in the Tetrahymena pyriformis-Klebsiella pneumoniae assay.

**Figure 10** shows OD values of Klebsiella pneumoniae assays using Tetrahymena pyriformis (Tp). The rows of the two blocks (50'000-9'250 and 6'250-0) are numbered with A-P from top to bottom as shown in figure 1. The rows A to P (1-12) are wild type strain Kp52145 (BAT39).

**Figure 11** shows OD values of Klebsiella pneumoniae assays using Tetrahymena thermophila (Tt). The rows of the two blocks (50'000-9'250 and 6'250-0) are numbered with A-P from top to bottom as shown in figure 1. The rows A to P (1-12) are wild type strain Kp52145 (BAT39).

**Figure 12** shows survival curves of mice infected with Klebsiella mutants in a pulmonary model of infection.

**Figure 13** shows the in vivo efficacy of 3-(allylsulfanyl)-5-hydroxy-6-methyl-1,2,4-triazine in Klebsiella pneumoniae murine pulmonary infection model.

**Detailed description of the invention**

As used herein, each of the following terms has the meaning associated with it in this section.

The term "infectivity", as used herein, is defined as the capability of a pathogen to cause disease, damage or harm to a host organism.

The term "anti-infective compound", as used herein, is intended to include compounds that reduce the virulence of a pathogen to a host organism and compounds that inhibit the growth of or kill a pathogen in the presence and/or in the absence of a host organism like antibiotic compounds.

The term "virulence", as used herein, is a measure of the degree of pathogenicity of a pathogen to a host organism. Virulence is usually expressed as the dose or cell number of a pathogen that will elicit a pathological response in the host organism within a given time period.

The term "anti-virulence activity", as used herein, is a measure of the degree of a compound that reduces the virulence of a pathogen to a host organism or a measure of the degree of a mutation of a mutant of a pathogen, which causes a reduced virulence to a host organism compared to the pathogen.

The term "virulence factor", as used herein, may be a poisonous substance, e. g., a toxin, a protein or a structure of the pathogen, that is produced by pathogenic living cells or organisms which initiates the pathogenic process or is required for initiating the pathogenic process and is capable of causing disease to a host.

The term "reducing the virulence", as used herein, is defined as the ability of a compound to attenuate, diminish, decrease, suppress, or arrest the development of, or the progression of disease, damage or harm to a host organism mediated by a pathogen.

The term "antibiotic activity", as used herein, is a measure of the degree of a compound that inhibit the growth of or kill a pathogen in the presence and/or in the absence of a host organism.

The term "quantifying a mutation in a virulence factor of a pathogen of a host organism", as used herein, is defined as the quantification of the phenotype resulting from a mutation in a virulence factor of a pathogen of a host organism.

The term "$IC_{50}$ (inhibitory concentration 50%)", as used herein, is defined as the concentration of an anti-infective compound which inhibits the growth of a pathogen by 50 %.

The term "host organism", as used herein, is intended to include any living organism. Preferably the host organism is a vertebrate. More preferably the host organism is a mammal. It is most preferred that the host organism is a human.

A "unicellular test organism", as used herein, is usually any living phagocytic protozoa, including, but not limited to, amoebae such as Dictyostelium, flagellates such as Bodo Saltans, ciliates such as Tetrahymena spp.. Preferably the phagocytic protozoon is a phagocytic amoeba, flagellate or ciliate. More preferred is a phagocytic ciliate such as, Paramecium, Stentor, Vorticella or Tetrahymena. Tetrahymena is the most preferred phagocytic protozoa, in particular Tetrahymena pyriformis (Tp) or Tetrahymena thermophila (Tt), more particular Tetrahymena pyriformis (Tp). The strain of Tp is not critical to the methods of the present invention. Tp strains such as CCAP1630/21 or CCAP1630/ 1W from CCAP (Culture Collection of Algae and Protozoa, SAMS Research Services Ltd., Dunstaffnage Marine Laboratory, Dunbeg, Argyll, PA37 1QA, UK) can be used in the present invention. A preferred strain is CCAP1630/21.

The term "pathogen", as used herein, intended to include any living microorganisms such as bacteria, mycobacteria, fungi and unicellular eukaryotic organism, including wild types and mutants thereof, which cause disease or brings about damage or harm to a host organism. The term "pathogenicity", as used herein, is defined as the ability of a pathogen to cause disease, damage or harm to a host organism.

Usually the pathogen is selected from the group consisting of bacteria, mycobacteria, fungi and unicellular eukaryotic organisms. Bacterial and mycobacterial pathogens of the present invention may include Helicobacter pyloris, Borelia burgdorferi, Legionella pneumophilia, Mycobacteria sps (e.g. M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus influenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue,Leptospira, Rickettsia, Actinomyces israelli, and Salmonella spp., Pseudomonas, e.g., P. aeruginos., Escherichia spp., e.g., Escherichia coli, Pathogenic fungi of the present invention may include, e. g., Candida albicans. Pathogenic unicellular eukaryotic organisms of the present invention may include, e. g., Leishmania donovani.

The pathogen can be an intracellular pathogen of the unicellular test organism, such as an intracellularly growing bacterial pathogen e. g., select strains of Salmonella, Legionella, or Listeria, or mycobacterial pathogen, e. g., Mycobacterium tuberculosis. Alternatively, the pathogen may be an extracellular pathogen that grows outside of the unicellular test organism, such as a bacterial pathogen as Pseudomonas, e. g., P. aeruginosa, Klebsiella, e.g. Klebsiella pneumonia, Escherichia, e. g., Escherichia coli, Staphylococcus, e. g., Staphylococcus aureus, Vibrio, e. g., Vibrio cholera, or an extracellularly growing pathogenic fungi, e. g., Candida albicans. Depending on the conditions of exposition an extracellular pathogen that grows outside of the unicellular test organism at the beginning of the exposition might become an intracellularly growing bacterial pathogen during exposition to the the unicellular test organism and vice versa.

Preferably the pathogen is a bacterial pathogen More preferably, the pathogen is an extracellular bacterial pathogen. Most preferably, the extracellular pathogen is Pseudomonas, Escherichia or Klebsiella. In particular the extracellular pathogen is selected from the group of Klebsiella pneumoniae, Pseudomonas aeruginosa, and Escherichia coli.

The virulence of the pathogen might be triggered by the quorum sensing pathway which is used by a broad range of pathogenic bacteria e. g., P. aeruginosa, Escherichia coli, Klebsiella pneumoniae, Staphylococcus aureus and Vibrio cholera, whereby a threshold concentration of autoinducer triggers the coordinated synthesis of secreted virulence factors which initiates the pathogenic process.

EXPOSING A UNICELLULAR TEST ORGANISM TO A PATHOGEN

In general the order of exposing the unicellular test organism to the pathogen in the absence or in the presence of the compound to be identified and/or quantified is not critical. Usually the unicellular test organism is exposed to a pathogen in the absence or in the presence of the anti-infective compound to be identified and/or quantified by adding cells from a culture of the pathogen to cells from a culture of the unicellular test organism. Exposition can be performed by adding the cells suspended in the medium used for culture or the cells can be suspended in other media or combinations thereof which allow for growth of the organisms during exposition to each other. Usually, cells from the culture are diluted with the culture medium used before being adding to each other. Preferably, the unicellular test organism is exposed to the pathogen in step (a), step (c) and step (d) of the method for identifying and/or quantifying the anti-infective compound under the same conditions. Exposition under the same conditions is performed by exposing cells of the pathogen to cells of the unicellular test organism in step (c) and step (d) suspended in the same medium or combination of media as used for exposition in step a).

Exposition can be performed on agar plates, on surfaces as glass cover slips, in tissue culture flasks, in test-tubes or on microplates. Preferably microplates which allow for visual and spectroscopic examination are used, more preferably such microplates which can be used for High Throughput Screening (HTS) are used.

Generally unstarved unicellular test organism cells are used in the methods of the present invention. For example, a test host organism can be cultured in adherent culture or in suspension culture for use in the methods of the present invention. In order to obtain a maximum reproducibility of the method, the test host organism is preferably cultured in suspension using a fermentor. Preferably the fermentor is continuously fed with medium whereas the culture should not exceed a maximum life span of one month. There is an optimum concentration of cultured cells of the test host organism in the fermentor for use in the method of the present invention which might vary depending on the particular host organism used and which can be assessed by determining the ratio of the concentration of the unicellular test organism to that of the pathogen which allows the pathogen to grow in the presence of the unicellular test organism. In case Tetrahymena such as Tetrahymena pyriformis (Tp) is used, the usual concentration is between 250'000 Tp/ml to 400'000 Tp/ml, the preferred optimum concentration is around 300'000 Tp/ml.

Any culture medium that will sustain the growth of the unicellular test organism may be used to produce the cells. SM medium as described in example 1 is preferably useful for growing the cells of the unicellular test organism for use in the present invention.

The conditions of pathogen culture are in general not critical to the methods of the present invention. Such conditions may be tailored to the pathogen being tested and are described in the art, e.g., for bacteria in Bergey's Manual of Systematic Bacteriology (The Prokaryotes 2nd edition by A. Balows etal., 1992). Under conditions where the virulence of the pathogen is triggered by the quorum sensing pathway, the pathogen is grown to a cell density at which the induction of the quorum sensing is maximal.

Usually one unicellular test organism is exposed to one pathogen. In order to identify and/or to quantify the anti-infective activity of an anti-infective compound as well two or more pathogens might be exposed simultaneously to the unicellular test organism in step c) of the present method, whereas usually the growth of one pathogen is monitored in step e).

Under certain conditions of exposition, the unicellular test organism may use a pathogen as a growth substrate. The ability of the unicellular test organism to grow on a pathogen substrate depends on the character of the pathogen, e. g., pathogen virulence. Other bacteria, e. g., E. coli or heat-killed bacteria may be added to a culture of the test host organism exposed to the pathogen and serve as a growth supplement for e.g. Tp. The growth supplement may be either a pathogenic or a non-pathogenic bacteria.

In order to determine the ratio of the concentration of the unicellular test organism to that of the pathogen which allows the pathogen to grow in the presence of the unicellular test organism in step b), usually one concentration of the pathogen is exposed to serial concentrations of the unicellular test organism cells in step a). The respective concentrations can be obtained by dilutions with the respective culture medium. Usually serial dilutions of the test organism to achieve a final cell concentration exposed to the pathogen of between 1000 and 100'000 cells/ml are used. The final cell concentration of the pathogen depends on the kind of pathogen. A bacterial pathogen is usually diluted to a cell concentration of between $10^3$ and $10^8$, preferably between 5x $10^4$ and 5x $10^6$ cells/ml, exposed to the unicellular test organism. The time for setting up the assay by adding the pathogen to different concentrations of the unicellular test organism cells should not exceed a period of 30 min in order to achieve a high reproducibility of the method. The concentration of the pathogen can be measured at the beginning of the exposition, but this is not required for the method. Usually corresponding dilutions of the pathogen without the unicellular test organism are set up simultaneously to and independently from the pathogen exposed to the unicellular test organisms at the beginning of the exposition in order to monitor the growth of the pathogen without the unicellular test organism.

After an exposition time of usually between 4 and 40, preferably between 8 and 35, more preferably between 15 and 30 hours, the growth of the pathogen at the different concentrations of the unicellular test organism is measured. The temperature at which the exposition is carried out depends on the unicellular test organism and on the pathogen used. Usually a temperature at which the growth of the pathogen is optimal is used. The choice of the temperature can be easily made by a person skilled in the art. The concentration of cells of the unicellular test organism at which the cells of the pathogen have completely overgrown the cells of the unicellular test organism at the end of the exposition time, is usually taken as the ratio of the concentration of the unicellular test organism to that of the pathogen which allows the pathogen to grow in the presence of the unicellular test organism. This concentration of cells of the unicellular test organism is referred herein as well as "threshold" or "threshold concentration".

The growth of the pathogen at the different concentrations of the unicellular test organism is usually monitored visually, microscopically or by measuring the absorbance of the pathogen at an appropriate wavelength. In case absorbance of the pathogen is measured completely overgrowing of the cells of the unicellular test organism by the cells of the pathogen is obtained if absorbance of the pathogen exposed to the unicellular test organism reaches the same level as absorbance of the pathogen without the unicellular test organism.

The ratios of the concentration of the unicellular test organism to that of the pathogen at which the cells of the pathogen have completely overgrown the cells of the unicellular test organism depend on the unicellular test organism and the pathogen used as well as on the culture conditions of the unicellular test organism. Depending on the culture conditions and the period of culture of the unicellular test organism the ratio can change within about 50 %.

Thus, the determination of the ratio is essential for calibrating the method of the present invention in order to achieve quantification and a high reproducibility. In case Tp is used as unicellular test organism and bacteria as pathogen, usual ranges for Tp are between 1000 and 100000 cells/ml at a cell concentration of the bacteria of between $10^3$ and $10^8$ cells/ml.

After Having determined the ratio of the concentration of the unicellular test organism to that of the pathogen which allows the pathogen to grow in the presence of the unicellular test organism, the unicellular test organism is exposed to the pathogen at the ratio determined in the absence and in the presence of the compound to be identified and/or quantified. Usually, cells of the unicellular test organism from the same culture, e. g. continous fermentor culture as described above, are used for determining the ratio of the concentration of the unicellular test organism to that of the pathogen which allows the pathogen to grow in the presence of the unicellular test organism in step b) and for carrying out step c)-d). In order for the ratio obtained in step b) to be usable for steps c)-d), the time period for determining the ratio of the concentration of the unicellular test organism to that of the pathogen which allows the pathogen to grow in the presence of the unicellular test organism in step b) and for carrying out step c)-d) usually should not exceed three days, preferably it should not exceed one day (24 hours). Culture conditions and exposition of the unicellular test organism and the pathogen in the absence and in the presence of the compound to be identified and/or quantified can be carried out as described supra. In the presence of the compound to be identified and/or quantified usually one concentration of the compound to be tested is added to the concentration of the unicellular test organism determined in step b) of the present method and then the corresponding concentration of cells of the pathogen is added to obtain the ratio of the concentration of the unicellular test organism to that of the pathogen which allows the pathogen to grow in the presence of the unicellular test organism. The unicellular test organism can be exposed in step c) to the pathogen as well independently in the presence of two or more concentrations of the compound to be identified and/or quantified, whereas in step e) the growth is monitored independently in the presence of two or more concentrations of the compound to be identified and/or quantified. The order of unicellular test organism, compound and pathogen added is in general not critical to the method of the invention. Usually cells from a culture of the pathogen are added to cells from a culture of the unicellular test organism and for the presence of the compound, the compound is added to the mixture of cells of the pathogen and of cells of the unicellular test organism.

In a preferred embodiment corresponding dilutions of the pathogen without the unicellular test organism are set up simultaneously to and under the same conditions of step c) and d) in order to monitor the growth of the pathogen in the presence and in the absence of the anti-infective compound without the unicellular test organism as described for step e). The level of growth obtained for the pathogen alone can be used to calculate the antibiotic activity of an anti-infective compound and to discriminate antivirulence activity from antibiotic activity as described below.

In a preferred embodiment of the present method the unicellular test organism is exposed in step c) at the ratio determined in step (b) and independently at one or more concentrations to the pathogen in the presence of the anti-infective compound to be identified and/or quantified. Usually two to four concentrations of the unicellular test organism higher and five to ten concentrations of the unicellular test organism lower than the concentrations determined in step b) is used. Preferably serial concentrations of between 100 000 unicellular cells and 0 cells are used. This preferred embodiment of the present method is particular sensitive for quantifying the anti-infective activity of the compound by quantifying the anti-infective activity of the compound with decreasing cell concentrations of the unicellular organism. The less ceiis of the unicellular organism the higher the anti-infective activity of the compound needs to be for the unicellular organism to eradicate, i. e. that is when no cells of the bacteria are present outside the unicellular organism anymore, the pathogen.

METHODS OF MONITORING THE GROWTH OF THE PATHOGEN

The pathogen growth may be monitored in many ways known to one skilled in the art. How cell growth is monitored depends, in part, on the method of pathogen exposure used. If the unicellular test organism and pathogen are exposed on agar plates or on surfaces such as glass cover slips, the agar plate or these surfaces may be incubated under conditions suited to growth and measurement (e. g., occurrence, number) of the pathogen. After the incubation period the occurrence and number of the pathogen in the presence of the compound are recorded and compared with the occurrence and number formed in the absence of the test compound. The number of pathogen and/or the colonies may be measured by any suitable means known to the person skilled in the art. Growth of the pathogen may be monitored e. g. fluorimetrically by well-known analytical techniques quantifying the total fluorescence emitted by a culture of the pathogen. It is common knowledge in the art that the level of fluorescence in culture test samples is directly proportional to the rate of cell growth. Growth of the pathogen can be monitored as well by measuring the absorbance (optical density) of the pathogen in the presence and in the absence of the anti-infective compound to be identified and/or quantified at an appropriate wavelength, wherein the difference between the absorbance of the pathogen measured in the presence of the anti-infective compound to be identified and/or quantified and the absorbance of the pathogen measured in the absence of the anti-infective compound to be identified and/or quantified quantifies the anti-infective activity of the compound. Measuring the absorbance of the pathogen is the preferred method of monitoring growth of the pathogen.

Usually, a wavelength is used at which absorbance of the cells of the pathogen is maximal and absorbance of the cells of the host organism is minimal or zero. In case of bacterial pathogens, the appropriate wavelength is usually at 250 to 600 nm, preferably at 450 nm. In case absorbance is used to monitor growth of the pathogen the anti-infective activity (AI) of the compound can be calculated as follows: The OD values measured at the beginning of the exposure are subtracted from the OD values measured at the end of the exposure in the presence and in the absence of the compound. These corrected values AIwt (for OD in the absence of the compound) and AI (for OD in the presence of the compound) are used to calculate the anti-infective activity of the compound using the formula AI%=100 x (1-(AI/AIwt)). Depending on the mode of action of the anti-infective compound (antivirulent or antibiotic) the same calculation can be performed for quantifying the antibiotic activity (AB) of a compound or the antivirulent activity (AV) of a compound.

Anti-infective activity of the compound can be quantified as 100% when the pathogen in the presence of the compound is completely eradicated, i. e. that is when no cells of the bacteria are present outside the unicellular organism anymore, by the unicellular organism and as 0% when the pathogen in the presence of the compound has the same level of growth as the pathogen in the absence of the compound and both completely overgrew the unicellular organism.

A further object of the present invention is the quantification of $IC_{50}$ of an anti-infective compound. Suitably, the method is a method for quantifying $IC_{50}$ of an anti-infective compound, as described above.

In case absorbance is used to monitor growth of the pathogen the $IC_{50}$ can be calculated by calculating the anti-infective activity, which is in this case equivalent to the anti-virulence activity (AV) of the compound as follows: The OD values measured at the beginning of the exposure are subtracted from the OD values measured at the end of the exposure in the presence and in the absence of the compound. These corrected values AVwt (for OD in the absence of the compound) and AV (for OD in the presence of two or more concentrations of the compound) are used to calculate the anti-infective activity of the compound using the formula for every concentration of the anti-infective compound AV%=100 x (1-($AV_{conc}$/AVwt)), Usually the AV values obtained are fed to a sigmoid approximation software and the resulting $IC_{50}$ values are computed and averaged using methods known to the person skilled in the art.

Usually the unicellular test organism is exposed to the pathogen at the ratio determined in step (b) independently in the presence of four to six concentrations, preferably four concentration of the anti-infective compound.

Preferably the unicellular test organism is exposed to the pathogen in step c) in addition independently in the presence of a standard compound and the growth of the pathogen is monitored in step (e) independently, in the presence of two or more concentrations of the anti-infective compound, in the absence of the anti-infective compound and in the presence of a standard compound, and wherein the level of growth of the pathogen in the presence of two or more concentrations of the anti-infective compound, the level of growth of the pathogen in the absence of the anti-infective compound and the level of growth in the presence of a standard compound are used to calculate the $IC_{50}$ of the anti-infective compound.

ANTI-INFECTIVE COMPOUNDS

Anti-infective compounds of the present invention include compounds that reduce the virulence of a pathogen to a host organism and compounds that inhibit the growth of or kill a pathogen in the presence and/or in the absence of a host organism like antibiotic compounds. Usually anti-infective compounds, which are identified and/or quantified by the present method are compounds that reduce the virulence of a pathogen to a host organism and the anti-infective activity of the compound is the anti-virulence activity of the compound. Anti-infective compounds, which are identified and/or quantified by the present method can be as well antibiotic compounds that inhibit the growth of or kill a pathogen in the presence and/or in the absence of a host organism and the anti-infective activity of the compound is the antibiotic activity of the compound. The present method can be used to discriminate between the anti-virulence activity and the antibiotic activity of an anti-infective compound. Anti-infective compounds of the present invention can include any synthetic or semi-synthetic compound. Such compounds include inorganic as well as organic chemical compounds. The compounds may be as well naturally occurring compounds. Naturally occurring compounds may include, e. g., saccharides, lipids, peptides, proteins, nucleic acids, or combinations thereof, e. g., aminoglycosides, glycolipids, lipopolysaccharides, or macrolides.

The precise source of the compound is not critical to the method of the present invention. The compound might be derived from e. g., synthetic compounds libraries which are commercially available, e. g., Sigma-Aldrich (Milwaukee, WI), or libraries of natural occurring compounds in the form of bacterial, fungal, plant, and animal extracts such as those available from Xenova (Slough,UK) might be chemically synthesized or produced by recombinant technologies according to methods known to the person skilled in the art. The synthetic (or semi-synthetic), natural occurring compounds or bacterial, fungal, plant, and animal extracts might be modified using standard chemical, physical, or biochemical methods known in the art. Usually one compound is used in the method of the present invention. The unicellular test organism can be exposed to the pathogen in the present method as well in the presence of two or more anti-infective compounds simultaneously e.g. in order to identify and/or quantify the synergistic effect of two or more anti-infective compounds.

The anti-infective compound to be identified and/or quantified by the method of the present invention may be produced in the course of exposing the unicellular test organism to a pathogen by another pathogen present in the assay mixture as long as the pathogen producing the compound does not grow during exposition. Alternatively, unicellular test organism cells may be preincubated with compound-producing pathogen cells prior to exposure to another pathogen used in the method of the invention that does not produce the compound or the compound might be produced by a pathogen culture in the absence of the unicellular test organism and the supernatant containing the compound might be used.

For identifying an anti-effective compound the monitored level of growth of the pathogen the presence of the compound to be identified and/or quantified has to be usually equal or less than 70 %, preferably equal or less than 50 %, more preferably equal or less than 30 %, most preferably equal or less than 10 % of the level of growth in the absence of the compound to be identified and/or quantified.

Animal experiments can be used to corroborate anti-virulence activity observed in the methods of the present invention. Thus, preferably the anti-virulence activity of the compound obtained with the method of the present invention corresponds to the and-virulence activity of the compound obtained in animal experiments. As animal experiments rodent mortality assays such as that described by Lavender et al. (2005), Int, J. Med. Micribiol. 295(3): 153-159, can be used. Preferably rodents, more preferably mice are used as animals to corroborate anti-virulence activity observed in the methods of the present invention.

Yet another object of the present invention is to provide a method for identifying and/or quantifying a mutation in a virulence factor of a pathogen of a host organism that reduces the virulence of the pathogen to a unicellular test organism. Such a method is described above.

Steps c) - g) of this method of the present invention can be carried out identically as steps a) - e) of the method for identifying and/or quantifying an anti-infective compound described supra except that no compound is present and that instead of exposing the unicellular test organism to the pathogen in the presence of the compound to be identified and/or quantified, the unicellular test organism is exposed to a mutant of the pathogen. The mutant of the pathogen is usually cultured and exposed to the unicellular test organism identically to the conditions described for the pathogen supra. As well the same methods of monitoring the growth of the pathogen as described above can be applied for monitoring the growth of the pathogen and the growth of the mutant of the pathogen. Usually, cells from the culture are diluted with the culture medium used before being adding to each other. Preferably, the unicellular test organism is exposed to the pathogen in step (c), step (e) and step (f) under the same conditions as defined supra. The unicellular test organism is preferably exposed to the pathogen in step f) independently to two or more mutants of the pathogen and the growth of the pathogen and of two or more mutants is monitored independently in step g). Anti-virulence activity of the mutant can be quantified as 100% when the pathogen mutant is completely eradicated i. e. that is when no cells of the bacteria are present outside the unicellular organism anymore, by the unicellular organism and as 0% when the pathogen mutant has the same level of growth as the pathogen and both completely overgrew the unicellular organism. Mutants with an anti-virulence activity of more than 30% are usually considered as less pathogenic, mutants with an anti-virulence activity of more than 50 % are usually considered as almost non-pathogenic, mutants with an anti-virulence activity of more than 75 % are usually considered as non-pathogenic. Usually the anti-virulence activity of the mutation of the mutant of the pathogen obtained with the method corresponds to the anti-virulence activity of the mutation of the mutant of the pathogen obtained in animal experiments. Preferably and-virulence activity greater than 75% obtained with the method of the present invention correlate at 100% with the anti-virulence activity of the mutation of the mutant of the pathogen obtained in animal experiments. Feasible animal experiments are as described supra.

Preferably, the growth of the pathogen and the growth of the mutant of the pathogen is monitored by measuring the absorbance of the pathogen and the absorbance of the mutant of the pathogen at an appropriate wavelength, wherein the difference between the absorbance of the pathogen and the absorbance of the mutant of the pathogen measured indicates the anti-virulence activity of the mutation of the mutant of the pathogen. Usually, a wavelength is used at which absorbance of the cells of the pathogen and of the mutant of the pathogen is maximal and absorbance of the cells of the host organism is minimal or zero. In case of bacterial pathogens, the appropriate wavelength is set at 250 to 600nm, preferably at 450 nm. In case absorbance is used to monitor growth of the pathogen and of the mutant of the pathogen the relative anti-virulence activity of the mutants can be calculated as follows: The OD values at the beginning of the exposure are subtracted from the OD values at the end of the exposure of the pathogen and of the mutant of the pathogen. These values AVwt (for OD the pathogen) and AV (for OD of the mutant of the pathogen) are used to calculate the antivirulent activity of the mutant of the pathogen using the formula $AV\%=100x(1-(AV/AVwt))$.

Preferably the unicellular test organism is exposed at the ratio determined in step (d) and independently at one or more concentrations to the pathogen and the mutant in steps e) and f) and wherein the growth of the pathogen and the growth of the mutant exposed at the ratio determined in step (d) and independently at one or more concentrations of the unicellular test organism is monitored in step g).

The pathogen which inhibits the growth of the unicellular test organism can be selected by the method for identifying and/or quantifying an anti-infective compound described supra. Any pathogen affecting the unicellular test organism growth may be mutated. Usually, the pathogen and its mutant is a bacterial pathogen. Preferably, the pathogen is an extracellular bacterial pathogen. More preferably, the pathogen is Pseudomonas, Escherichia or Klebsiella. Most preferably the pathogen is is selected from the group of Klebsiella pneumoniae, Pseudomonas aeruginosa and Escherichia coli.

The mutant of the pathogen can be selected according to known methods in the art such as ultraviolet radiation exposure, treatment with intercalating agent or transducing phage or a transposon insertion. On the other hand, mutants of the pathogen already known in the art can be selected. The mutation can be used as a marker employing methods known in the art to further identify the virulence factor and e. g. the respective genes encoding proteins required for infection of a host organism.

Thus the method for identifying and/or quantifying a mutation in a virulence factor of a pathogen of a host organism that reduces the virulence of the pathogen to a unicellular test organism of the present invention can be used to identify the gene or genes which have been mutated and which are encoding the virulence factors of a pathogens to a host organism.

For controlling quality and reproducibility of the methods of the present invention, the unicellular test organism, the pathogen, the mutant of the pathogen and/or the compound can be exposed and monitored alone or in various combinations simultaneously to the steps defined by the methods of the present invention, e. g. as internal controls, which can be performed simultaneously in the respective steps. If the methods of the present invention are carried out as HTS assay which is preferred, matrix microtiter plates, reduced matrix plates as well as quality control plates of the compound to be identified and/or quantified as described in example 2 can be used. As well the methods of the present invention can be carried out using one microtiter plate.

Statistical functions known to the person in the art to analyse HTS assays like the one described by Zhang et al., 1999, J. Biomol. Screen, 4, 67-73, can be used to evaluate the robustness of the method. In case the statistical function described by Zhang et al., 1999, J. Biomol. Screen, 4, 67-73, is used a Z-factor higher than 0.5, preferably higher than 0.8 the maximum value obtained so far can be achieved with the method of the present invention.

## Examples

### Example 1

### Threshold determination of Klebsiella

### Preparation of SM no glucose (SM-), and Tetrahymena medium

Procedure to prepare one liter of SM liquid medium:

| | |
|---|---|
| Bactopeptone [Oxoid L37] | 10.00 g |
| Yeast extract [Bacto LP0021B]] | 1.00 g |
| $KH_2PO_4$ [Fluka, 60230] | 2.20 g |
| $K_2HPO_4$ [Applichem A2945] | 1.00 g |
| $MgSO_4$ x $7H_2O$ [Aplichem A1037] | 1.00 g |

Weighting tolerance: ± 0.05 g

Sterilize by filtration using a 0.22 μm pores filter (Millipore, SCGPT05RE). Keep this medium in dark at room temperature no more than one week.

| | |
|---|---|
| Tetrahymena medium : | |
| Proteose peptone [Oxoid LP0085] | 5 g |
| Tryptone [Oxoid LP0042] | 5 g |
| KH2PO4 | 0.2 g |

Complete to 11 with bidistilled $H_2O$ (dd $H_2O$). Autoclave 20 minutes at 121 °C. Allow solutions to cool down to room temperature. Add 1ml of 0.22 μM filtered $FeCl_3$ $6H_2O$ at 110 μM.

**1. Strains description**

Klebsiella pneumoniae is an opportunistic pathogen that frequently causes nosocomial infections, mainly in immunocompromised patients. K. pneumoniae infections range from mild urinary infections to severe bacteremia and pneumonia with a high rate of mortality and morbidity. Pulmonary infections due to K. pneumoniae are often characterized by a rapid progressive clinical course complicated by lung abscesses and multilobular involvement which leaves little time to establish an effective antibiotic treatment. In this assay we put the virulent wild-type Kp 52145 strain (BAT39, Nassif X., Sansonetti P.J. (1986). Infect Immun. 54(3):603-8) with Tp cells in order to find compounds active against Kp pathogenicity.

**2. Preparation of Tetrahymena pyriformis (Tp) :**

**Preparation of Tp for transfer in the fermentor**

Every two weeks a new fermentor is started:

Tp cells (strain CCAP1630/21) from the 16°C stock is transferrer to fresh Tp medium, cultured at 32 °C for 5 days and transferred to the fermentor at a concentration of 300'000 Tp/ml, together with a magnet stirrer.

**Installation fermentor**

The fermentor is put on a stirrer in a 32°C cabinet, and the flowrate adjusted to get the desired cell density.

**Fermentor maintenance**

The cell density range should be between 250'000 Tp/ml to 400'000 Tp/ml and weekly maintenance involves to refill the medium supply.

**3. Threshold determination**

Cells are transferred into a 750 ml tissue culture flask and grown overnight culture at 32 °C. Tp are diluted in SM- to reach cell densities of 100'000, 74'000, 50'000, 37'000, 25'000, 18'500,12'500, 9'250, 6'250, 4'625, and 3'125 Tp/ml, to dispense the cells into the 384 well plates. DMSO is added with a 384 pin replicator.

**Preparation of Klebsiella pneumoniae (Kp)**

Final dilution of Kp for screening is obtained from overnight culture at 37°C and 250 rpm and bacteria are dispensed using a liquid dispenser.

**Incubation**

At 35 °C in humidity saturated incubator. Read endpoint at OD450 ηm after 22-30 hours of incubation. The threshold if readily established from the matrix plate: the screening Tp concentration shall be set at the least Tp concentration where the bacteria have overgrown Tp in a homogeneous manner.

**Example 2**

**High Throughput Screening**

1. Preparation of SM no glucose (SM-) and Tetrahymena Medium is as described in example 1.
2. Strains description
Same Klebsiella pneumoniae strain is used as in example 1.
3. Preparation of Tetrahymena pyriformis (Tp):

    The same Tp is used as in example 1. The day of the screen, resuspend the Tp cells in SM- and distribute Tp cells in the 384 well plates.

4. Liquid dispenser for medium distribution
Fill the same number of test plates as above with SM- with the liquid dispenser plate filler. (To test the antibiotic activity of the compounds).
5. Description of the mother plate of compounds
Each mother plate containing 24 columns of compounds is composed of 4 control columns ( 1, 12, 13 and 24) of DMSO and 320 wells of compounds. The DMSO columns have to be arranged according to the library.
6. Transfer of library compounds with the 384 pin replicator.
A test plate for quantifying anti-virulence activity contains Tp solution. A test plate for quantifying antibiotic activity contains SM-. Take the pin replicator and simply line up the guide pins in the alignment holes of the mother plate of compounds. The replicator pins will be aligned to the microplate wells. Wash the pin replicator and let it dry.
7. QC plates and controls in the reduced matrix
For the DMSO QC plate and the reduced matrix plate transfer DMSO is pin-replicated from a mother plate. Perform 3 QC plates, 1 at the start of the assay, one in the middle and one at the end.
8. Preparation of Klebsiella pneumoniae (Kp)
Klebsiella pneumoniae is prepared and dispense as in example 1.
9. Activity Calculation:
From the raw data (OD values), for each well substract time 0 values from time 1 values. Calculate the average OD value of the WT (wild type) without compound (AVwt from AV set and ABwt from AB set). Calculate the antivirulent activity (AV%) for each of the 320 compounds on each plate using the formula: AV%=100x(1-(AV/AVwt)). Calculate the antibiotic activity (AB%) using the test plate without Tp using the formula: AB%=100x(1-(AB/ABwt))
10. Z' factor calculation:
Z'-factor is a statistical function used commonly to judge high throughput screening assay robustness (Zhang,J. et al. (1999) J. Biomol. Screen. 4:67-73). Z'-factors greater that 0.50 are indicative of an excellent assay. For cell-based assays, it is common to use a Z'-factor greater than 0.4 as this indicator. The formula for Z'-factor takes into consideration standard deviation as well as the difference in the means of the high (untreated) and low (treated) values of an assay. It is calculated by the following formula:

$$Z' = 1 - (3\sigma_1 + 3\sigma_2) / |\mu_1 - \mu_2|$$

The Z' factor can be calculated for each assay plate if a control compound is added in the "control" row, or if a non-pathogenic mutant is used instead of wt.

**Example 3**

**High Throughput Screening Assay of anti-infective compounds and mutants**

1) Assays specifications

Bacteria and Tp concentrations were set at the established threshold obtained according to example 1 as can be seen from Table 1: Concentrations are given in cells/ml. Tp concentration could be varied from $10^3$ to $10^5$. Bacteria concentration could be varied from $10^4$ to $10^7$.

**Table 1**

| Bacteria | Assay medium | Average Tp number | Bacteria number | Endpoint time |
|---|---|---|---|---|
| Klebsiella pneumoniae | SM without glucose | 25000 | $5x10^6$ | 16-28h |
| Pseudomonas aeruginosa | TSB (CM0129) : Oxoid Ltd., England. | 9250 | $1x10^5$ | 16-28h |
| Escherichia coli | SM no glucose | 6250 | $5x10^5$ | 16-28h |

2) Tetrahymena growth phase

Cells are grown in a fermentor, as described in example 1, to a cell density ranging from 250'000 to 400'000 Tp/ml. The routinely used optimum concentration of Tetrahymena cells for High Throughput Screening (HTS) assays is around 300'000 Tp/ml.

3) Example of mutants and compound in the matrix of Tp concentration

a) Klebsiella pneumoniae, 8 mutants and one compound

Tp, Klebsiella pneumoniae, compound are prepared as described in example 2 and incubated in a matrix containing 384 wells (12 x 24) wells at 35°C in humidity saturated incubator. The mutants are prepared and distributed as described in example 2 for the wild type. The obtained matrix and corresponding OD values of the Klebsiella pneumoniae assay and the evaluation of the phenotype of the mutants and the potency of compound ATH18534 can be seen in Figure 1 and Figure 2. Tp concentrations are increasing from the right to the left, starting from zero cells/ml. The bacteria concentration was set to $5x10^6$ cell/ml. The threshold, where Tp cells are overgrown by bacteria was observed at 25,000 Tp/ml. The rows A, J to L and N to P are wild type strain Kp52145 (BAT39). Rows B to I are mutant strains, BAT21, BAT30, BAT27, BAT 146, BAT168, BAT172, BAT187 and BAT199, respectively. The row M is the wild-type strain treated with 10 μM of compound ATH18534 (3-(allylsulfanyl)-5-hydroxy-6-methyl-1,2,4-triazine). Mutations or compound effecting pathogenicity could be assessed in terms of phenotype strength and potency, respectively with decreasing concentration of Tp. Mutants BAT21 and BAT146 were permissive for growth of the Tp up to the lowest concentration of Tp. The less Tp the stronger the phenotype of the mutant and the more potent the compound need to be for Tp to grow and eradicate the bacteria.

Reduced matrix plates and quality control plates using DMSO solvent only were incubated at 35 °C simultaneously in humidity saturated incubator. Noise was determined using DMSO only in a plate containing Tetrahymena in columns 1 to 24 and bacteria in columns 2 to 12 and 14 to 24. A second plate contained only bacteria.

Figure 3 shows the antibiotic (abscissa) and avirulence (ordinate) activities of the screening plates determined according to the formula as described in example 2. The cloud of dots centered around the zero represents the noise of the assay. The few dots with avirulence activities higher than the noise represent the hits. Hits should have a antibiotic activity equal or smaller to the noise.

b) Uropathogenic E. coli (ATCC 700928) and 8 mutants

Tp, E. coli, compound and mutant are prepared as described in example 2 for Kp and incubated at 35°C in humidity saturated incubator on a matrix containing 384 wells (12 x 24) wells as desribed in 3a). The mutants are prepared and distributed as described in example 2 for the wild type. The obtained OD values of the E. coli assay and the evaluation of the phenotype of the mutants can be seen in Figure 4.

Tp concentrations are increasing from the right to the left, starting from zero cells/ml. The bacteria concentration was set to $5x10^5$ cell/ml. The threshold, where Tp cells are overgrown by bacteria was observed at 6,250 Tp/ml. The rows A, J to P are wild type strain BAT452. Rows B to I are mutant strains, BAT501, BAT502, BAT503, BAT 505, BAT546, BAT549, BAT550 and BAT563, respectively. Mutations affecting pathogenicity could be assessed in terms of phenotype strength with decreasing concentration of Tp. Mutants BAT501 and BAT549 were permissive for growth of the Tp up to the lowest concentration of Tp. The less Tp the stronger the phenotype of the mutant need to be for Tp to grow and eradicate the bacteria.

Reduced matrix plates and quality control plates using DMSO solvent only were incubated at 35°C simultaneously in humidity saturated incubator. Noise was determined using DMSO only in a plate containing Tetrahymena in columns 1 to 24 and bacteria in columns 2 to 12 and 14 to 24. A second plate contained only bacteria. Figure 5 shows the antibiotic (abscissa) and avirulence (ordinate) activities of the screening plates determined according to the formula as described in example 2. The cloud of dots centered around the zero represents the noise of the assay. The few dots with avirulence activities higher than the noise represent the hits. Hits should have a antibiotic activity equal or smaller to the noise.

c) Pseudomonas aeruginosa (BAT10, Cosson P., Zulianello L., Join-Lambert O., Faurisson F., Gebbie L., Benghezal M., Van Delden C., Curty L.K., Kohler T. (2002). J. Bacteriol. 184(11):3027-33.) and 8 mutants

Tp, Pseudomonas aeruginosa, compound and mutant are prepared as described in example 2 for Kp and incubated at 35 °C in humidity saturated incubator on a matrix containing 384 wells (12 x 24) wells as described in 3a). The mutants are prepared and distributed as described in example 2 for the wild type. The obtained OD values of the Pseudomonas aeruginosa assay and the evaluation of the phenotype of the mutants can be seen in Figure 6. Tp concentrations are increasing from the right to the left, starting from zero cells/ml. The bacteria concentration was set to $1x10^5$ cell/ml. The threshold, where Tp cells are overgrown by bacteria was observed in this particular case at 18,250 Tp/ml. The rows A, J to P are wild type strain BAT10. In row J bacteria were omitted for Tp concentration of 18,500 to 9,250 cells/ml). Rows B to I are mutant strains, BAT331, BAT2, BAT334, BAT357, BAT370, BAT375, BAT406 and BAT408, respectively. Mutations affecting pathogenicity could be assessed in terms of phenotype strength with decreasing concentration of Tp. Mutants BAT334 had the strongest phenotype.

Reduced matrix plates and quality control plates using DMSO solvent only were incubated at 35°C simultaneously in humidity saturated incubator. Noise was determined using DMSO only in a plate containing Tetrahymena in columns 1 to 24 and bacteria in columns 2 to 12 and 14 to 24. A second plate contained only bacteria. Figure 7 shows the antibiotic (abscissa) and avirulence (ordinate) activities of the screening plates determined according to the formula as described in example 2. The cloud of dots centered around the zero represents the noise of the assay. The few dots with avirulence activities higher than the noise represent the hits. Hits should have a antibiotic activity equal or smaller to the noise.

**Example 4**

**Measurement of $IC_{50}$ of ATH18534 (3-(allylsulfanyl)-5-hydroxy-6-methyl-1,2,4-triazine) in the Tetrahymena pyriformis-Klebsiella pneumoniae assay**

Assay conditions for Tetrahymena pyriformis-Klebsiella pneumoniae are as described in example 3,3 a. Each mother IC50 plate of compounds is composed of 4 control columns of DMSO (12 μl per well) and 32 wells per compound (12 μl per well) as can be seen from figure 8A. 4 replicates dose responses are tested per compound at different dose ranges as can be seen from figure 8B. Anti-virulence activity is calculated as follows: AV%=100x(1-(OD/ODwt)). ODwt was of 0.65. All 4 sigmoidal curves have been drawn on the same graph for each of the 10 compound, $IC_{50}$ values for all 4 curves were calculated as well as the average value and its standard deviation and $IC_{50}$ was determined using Prism version 4 software (Figure 8C).

**Example 5**

**Sub-MIC effect of Trimethoprim in the *Tetrahymena pyriformis- klebsiella pneumoniae* assay**

The effect of the trimethoprim antibiotic was quantified in the Tetrahymena-Klebsiella assay and virulence and antibiotic activities were calculated and compared as described in example 3, 3 a for different concentrations of trimethoprim. The avirulence assay detected the antibiotic at a concentration that was lower of a magnitude of 2 Log of the $IC_{50}$ of the antibiotic as can be seen in Figure 9. Thus the avirulence assay is highly sensitive to detect antibiotic activities at sub-MIC level.

**Example 6**

**Comparison of matrixes of Klebsiella pneumoniae assays using either Tetrahymena pyriformis (Tp) or Tetrahymena thermophila (Tt)**

Tp, Klebsiella pneumoniae, compound are prepared as described in example 2 and incubated in a matrix containing 384 wells (12 x 24) wells at 35 °C in humidity saturated incubator. Tt (CCAP 1630/1M) is prepared and distributed as described in example 2 for Tp. Cell concentrations are increasing in the matrix from the right to the left, starting from zero cells/ml. The bacteria concentration was set to $5x10^6$ cell/ml. The threshold for Tp was observed at 37,000 cells/ml and at 25,000 cells/ml for Tt as can be seen from figures 10 and 11. The rows A to P (1-12) are wild type strain Kp52145 (BAT39).

**Example 7**

**Validation of mutants scoring in the Tetrahymena assays**

**a) Klebsiella mutants validated in the mouse pneumoniae model**

Wild-type Klebsiella killed all the 6 mice in 75h (pathogenic), mutants leading to the death of one mouse were considered as nonpathogenic, mutants leading the deaths of 2 to 3 mice were considered as almost nonpathogenic and mutants leading to the deaths of 5 mice were considered as less pathogenic.

The protocol was approved by the Swiss federal veterinary office (authorization number 31.1.1083/2175/III). Bacteria were grown on a TSB Petri dish for 15h at 37°C. They were then scraped from the Petri dish and resuspended in sterile 0.9% NaCl. 7-8 weeks old female Balb/cJ mice (6 for each strain tested) were anaesthetized with ketamine (40 mg/kg) and xylazine (5 mg/kg), infected with 106 bacteria by nasal instillation of 20 μl and tagged with a transponder for identification and body temperature measurement twice a day. A body temperature below 34°C or a loss of more than 20% of body weight were found to be predictive of the death of the mice in the next 15 hours. These criteria were used as endpoints for euthanasia. Survival curves of mice infected with Klebsiella mutants in a pulmonary model of infection are shown in Figure 12.

**b) Activity of Klebsiella mutants in the Tp assay**

Klebsiella mutants tested in a pulmonary model of infection showed an attenuated pathogenicity, ranging from a slight attenuation to complete loss of pathogenicity. Avirulence activity in the Tp assay as desribed in example 33a) was defined as 100% when bacteria were completely eradicated by Tp and as 0% when bacteria overgrew the Tp. Mutants with an activity of more that 30% were considered as a hit in the Tp assay as can be seen from Table 2.

| Mutant | Avirulence activity (%) | In vivo evaluation |
|---|---|---|
| BAT39 | 0.00 | pathogenic |
| BAT176 | 22.72 | pathogenic |
| BAT164 | 28.54 | Less pathogenic |
| BAT200 | 40.35 | Almost nonpathogenic |
| BAT153 | 80.93 | Nonpathogenic |

**Example 8**

**In vivo efficacy of 3-(allylsulfanyl)-5-hydroxy-6-methyl-1,2,4-triazine in Kp murine pulmonary infection model.**

Klebsiella pulmonary infection model was performed by intranasal instillation of 7-8 weeks old female Balb/cN with 20 μl containing 106 bacteria. Compound or cefotaxime were injected intraperitoneally every 6 hours from the time of infection at the indicated doses. In vivo efficacy was assessed by measuring colony forming units (CFU) loads in the lungs at 24h hours. Data analysis was performed using GraphPad Software Inc, San Diego, California, USA. The results are shown in Figure 13. Lane 1 is the initial load at time zero of the infection. Lane 2 is the load at 24hours post-infection of mice treated with the vehicle only. Lane 3 is the load at 24hours post-infection of mice treated cefotaxime at 37.5 mg/kg/day and lane 4 is the load at 24hours post-infection of mice treated with [3-(allylsulfanyl)-5-hydroxy-6-methyl-1,2,4-triazine] at 225 mg/kg/day. [3-(allylsulfanyl)-5-hydroxy-6-methyl-1,2,4-triazine] and cefotaxime antibiotic treatments reduced the CFU load in the lungs by a magnitude of 4 Log.

## Claims

1. A method for identifying and/or quantifying the anti-infective activity of an anti-infective compound, said method comprising:

    (a) exposing an unicellular test organism to a pathogen of a host organism in the absence of the anti-infective compound to be identified and/or quantified;
    (b) determining the ratio of the concentration of the unicellular test organism to that of the pathogen which allows the pathogen to grow in the presence of the unicellular test organism;
    (c) exposing the unicellular test organism to the pathogen at the ratio determined in step (b) in the presence of the anti-infective compound to be identified and/or quantified;
    (d) exposing the unicellular test organism to the pathogen at the ratio determined in step (b) in the absence of the anti-infective compound to be identified and/or quantified; and
    (e) monitoring the growth of the pathogen in the presence and in the absence of the anti-infective compound to be identified and/or quantified, wherein a lower level of growth of the pathogen in the presence of the anti-infective compound to be identified and/or quantified than observed in the absence of the anti-infective compound to be identified and/or quantified indicates that the anti-infective compound to be identified and/or quantified has an anti-infective activity.

2. The method of claim 1, wherein the unicellular test organism is exposed in step (c) at the ratio determined in step (b) and independently at one or more concentrations to the pathogen in the presence of the anti-infective compound to be identified and/or quantified.

**3.** The method of claim 1 or claim 2, wherein simultaneously to and under the same conditions of step c) and d) the pathogen is exposed without the unicellular test organism in the presence and in the absence of the anti-infective compound to be identified and/or quantified, and the growth of the pathogen in the presence and in the absence of the anti-infective compound to be identified and/or quantified is monitored corresponding to step (d), wherein the level of growth obtained for the pathogen in the presence and in the absence of the anti-infective compound to be identified and/or quantified is used to calculate the antibiotic activity of the anti-infective compound and to discriminate anti virulence activity from antibiotic activity of the anti-infective compound.

**4.** The method of any one of claims 1-3, wherein the anti-infective compound to be identified and/or quantified is selected from

(1) a compound that reduces the virulence of a pathogen to a host organism and the anti-infective activity of the compound is the anti-virulence activity of the compound, and
(2) an antibiotic compound that inhibits the growth of or kills a pathogen in the presence and/or in the absence of a host organism and the anti-infective activity of the compound is the antibiotic activity of the compound.

**5.** The method according to any one of claims 1 to 4, wherein the method is a method for quantifying $IC_{50}$ of an anti-infective compound, wherein steps (c) to (e) of the method are as follows:

(c) exposing the unicellular test organism to the pathogen at the ratio determined in step (b) independently in the presence of two or more concentrations of the anti-infective compound;
(d) exposing the unicellular test organism to the pathogen at the ratio determined in step (b) in the absence of the anti-infective compound; and
(e) monitoring the growth of the pathogen independently in the presence of two or more concentrations of the anti-infective compound and in the absence of the anti-infective compound, wherein the level of growth of the pathogen in the presence of two or more concentrations of the anti-infective compound and the level of growth of the pathogen in the absence of the anti-infective compound are used to calculate the $IC_{50}$ of the anti-infective compound.

**6.** The method of claim 5, wherein the unicellular test organism is exposed to the pathogen in step c) in addition independently in the presence of a standard compound and the growth of the pathogen is monitored in step (e) independently, in the presence of two or more concentrations of the anti-infective compound, in the absence of the anti-infective compound and in the presence of the standard compound, and wherein the level of growth of the pathogen in the presence of two or more concentrations of the anti-infective compound, the level of growth of the pathogen in the absence of the anti-infective compound and the level of growth in the presence of the standard compound are used to calculate the $IC_{50}$ of the anti-infective compound.

**7.** A method for identifying and/or quantifying a mutation in a virulence factor of a pathogen of a host organism that reduces the virulence of the pathogen to a unicellular test organism, said method comprising:

(a) selecting a pathogen of a host, wherein the pathogen is able to grow in the presence of the unicellular test organism;
(b) selecting a mutant of the pathogen;
(c) exposing the unicellular test organism to the pathogen;
(d) determining the ratio of the concentration of the unicellular test organism to that of the pathogen which allows the pathogen to grow in the presence of the unicellular test organism;
(e) exposing the unicellular test organism to the pathogen at the ratio determined in step (d);
f) exposing the unicellular test organism to the mutant of the pathogen at the ratio determined in step (d); and
g) monitoring the growth of the pathogen and the growth of the mutant of the pathogen, wherein a higher level of growth of the pathogen than observed for the mutant of the pathogen indicates that the mutant of the pathogen has a mutation in the virulence factor of the pathogen that reduces the virulence of the pathogen to the host organism.

**8.** The method of claim 7, wherein the unicellular test organism is exposed at the ratio determined in step (d) and independently at one or more concentrations to the pathogen and the mutant in steps e) and f) and wherein the growth of the pathogen and the growth of the mutant exposed at the ratio determined in step (d) and independently at one or more concentrations of the unicellular test organism is monitored in step g).

**9.** The method of any one of claims 1-8, wherein the unicellular test organism is a phagocytic protozoa optionally selected from a phagocytic amoeba, flagellate and ciliate.

**10.** The method of claim 9, wherein the unicellular test organism is Tetrahymena.

**11.** The method of claim 10, wherein Tetrahymena is Tetrahymena pyriformis or Tetrahymena thermophila.

**12.** The method of any one of claims 1-11, wherein the pathogen is selected from the group consisting of bacteria, mycobacteria, fungi and unicellular eukaryotic organisms.

**13.** The method of claim 12, wherein the pathogen is an extracellular bacterial pathogen.

**14.** The method of claim 13, wherein the extracellular bacterial pathogen is selected from the group consisting of Klebsiella pneumoniea, Pseudomonas aeruginosa and Escherichia coli.

**15.** The method of any one of claims 1-14, wherein the host organism is a vertebrate.

**Patentansprüche**

**1.** Verfahren zum Identifizieren und/oder Quantifizieren der infektionshemmenden Wirkung einer infektionshemmenden Verbindung, welches Verfahren Folgendes umfasst:

(a) das Exponieren eines einzelligen Testorganismus gegenüber einem Pathogen eines Wirtsorganismus in Abwesenheit der zu identifizierenden und/oder zu quantifizierenden infektionshemmenden Verbindung;
(b) das Bestimmen des Verhältnisses der Konzentration des einzelligen Testorganismus zu jener des Pathogens, das es dem Pathogen ermöglicht, in Gegenwart des einzelligen Testorganismus zu wachsen;
(c) das Exponieren des einzelligen Testorganismus gegenüber dem Pathogen bei dem in Schritt (b) bestimmten Verhältnis in Gegenwart der zu identifizierenden und/oder zu quantifizierenden infektionshemmenden Verbindung;
(d) das Exponieren des einzelligen Testorganismus gegenüber dem Pathogen bei dem in Schritt (b) bestimmten Verhältnis in Abwesenheit der zu identifizierenden und/oder zu quantifizierenden infektionshemmenden Verbindung;
(e) das Beobachten des Wachstums des Pathogens in Gegenwart und in Abwesenheit der zu identifizierenden und/oder zu quantifizierenden infektionshemmenden Verbindung, worin ein geringeres Wachstumsausmaß des Pathogens in Gegenwart der zu identifizierenden und/oder zu quantifizierenden infektionshemmenden Verbindung als es in Abwesenheit der zu identifizierenden und/oder zu quantifizierenden infektionshemmenden Verbindung beobachtet wird ein Indikator dafür ist, dass die zu identifizierende und/oder zu quantifizierende infektionshemmende Verbindung eine infektionshemmende Wirkung aufweist.

**2.** Verfahren nach Anspruch 1, worin der einzellige Testorganismus in Schritt (c) bei dem in Schritt (b) bestimmten Verhältnis und unabhängig davon bei einer oder mehreren Konzentrationen gegenüber dem Pathogen in Gegenwart der zu identifizierenden und/oder zu quantifizierenden infektionshemmenden Verbindung exponiert wird.

**3.** Verfahren nach Anspruch 1 oder Anspruch 2, worin gleichzeitig zu und unter den gleichen Bedingungen von Schritt (c) und Schritt (d) das Pathogen ohne den einzelligen Testorganismus in Gegenwart und in Abwesenheit der zu identifizierenden und/oder zu quantifizierenden infektionshemmenden Verbindung exponiert wird und das Wachstum des Pathogens in Gegenwart und in Abwesenheit der zu identifizierenden und/oder zu quantifizierenden infektionshemmenden Verbindung gemäß Schritt (d) beobachtet wird, worin das für das Pathogen in Gegenwart und Abwesenheit der zu identifizierenden und/oder zu quantifizierenden infektionshemmenden Verbindung erhaltene Wachstumsausmaß verwendet wird, um die antibiotische Wirkung der infektionshemmenden Verbindung zu berechnen und um die virulenzhemmende Wirkung von der antibiotischen Wirkung der infektionshemmenden Verbindung zu unterscheiden.

**4.** Verfahren nach einem der Ansprüche 1 bis 3, worin die zu identifizierende und/oder zu quantifizierende infektionshemmende Verbindung aus

(1) einer Verbindung, die die Virulenz eines Pathogens auf einen Wirtsorganismus reduziert, wobei die infektionshemmende Wirkung der Verbindung die virulenzhemmende Wirkung der Verbindung ist, und
(2) einer antibiotischen Verbindung, die in Gegenwart und/oder in Abwesenheit eines Wirtsorganismus das Wachstum eines Pathogens hemmt oder dieses tötet, wobei die infektionshemmende Wirkung der Verbindung die antibiotische Wirkung der Verbindung ist,

ausgewählt ist.

**5.** Verfahren nach einem der Ansprüche 1 bis 4, worin das Verfahren ein Verfahren zum Quantifizieren des $IC_{50}$ einer infektionshemmenden Verbindung ist, worin die Schritte (c) bis (e) des Verfahrens wie folgt lauten:

(c) das Exponieren des einzelligen Testorganismus gegenüber dem Pathogen bei dem in Schritt (b) bestimmten Verhältnis unabhängig voneinander in Gegenwart zweier oder mehrerer Konzentrationen der infektionshemmenden Verbindung;
(d) das Exponieren des einzelligen Testorganismus gegenüber dem Pathogen bei dem in Schritt (b) bestimmten Verhältnis in Abwesenheit der infektionshemmenden Verbindung; und
(e) das Beobachten des Wachstums des Pathogens unabhängig voneinander in Gegenwart zweier oder mehreren Konzentrationen der infektionshemmenden Verbindung und in Abwesenheit der infektionshemmenden Verbindung, worin das Wachstumsausmaß des Pathogens in Gegenwart zweier oder mehrerer Konzentrationen der infektionshemmenden Verbindung und das Wachstumsausmaß des Pathogens in Abwesenheit der infektionshemmenden Verbindung verwendet werden, um den $IC_{50}$ der infektionshemmenden Verbindung zu berechnen.

**6.** Verfahren nach Anspruch 5, worin der einzellige Testorganismus gegenüber dem Pathogen in Schritt (c) zusätzlich dazu unabhängig davon in Gegenwart einer Standardverbindung exponiert wird und das Wachstum des Pathogens in Schritt (e) unabhängig voneinander in Gegenwart zweier oder mehrerer Konzentrationen der infektionshemmenden Verbindung, in Abwesenheit der infektionshemmenden Verbindung und in Gegenwart der Standardverbindung beobachtet wird und worin das Wachstumsausmaß des Pathogens in Gegenwart zweier oder mehrerer Konzentrationen der infektionshemmenden Verbindung, das Wachstumsausmaß des Pathogens in Abwesenheit der infektionshemmenden Verbindung und das Wachstumsausmaß in Gegenwart der Standardverbindung verwendet werden, um den $IC_{50}$ der infektionshemmenden Verbindung zu berechnen.

**7.** Verfahren zum Identifizieren und/oder Quantifizieren einer Mutation in einem Virulenzfaktor eines Pathogens eines Wirtsorganismus, die die Virulenz des Pathogens auf einen einzelligen Testorganismus reduziert, welches Verfahren Folgendes umfasst:

(a) das Auswählen eines Pathogens eines Wirts, worin das Pathogen in der Lage ist, in Gegenwart des einzelligen Testorganismus zu wachsen;
(b) das Auswählen einer Mutante des Pathogens;
(c) das Exponieren des einzelligen Testorganismus gegenüber dem Pathogen;
(d) das Bestimmen des Verhältnisses der Konzentration des einzelligen Testorganismus zu jener des Pathogens, das es dem Pathogen ermöglicht, in Gegenwart des einzelligen Testorganismus zu wachsen:
(e) das Exponieren des einzelligen Testorganismus gegenüber dem Pathogen bei dem in Schritt (d) bestimmten Verhältnis;
(f) das Exponieren des einzelligen Testorganismus gegenüber der Mutante der Pathogens bei dem in Schritt (d) bestimmten Verhältnis; und
(g) das Beobachten des Wachstums des Pathogens und des Wachstums der Mutante des Pathogens, worin ein höheres Wachstumsausmaß des Pathogens als das bei der Mutante des Pathogens beobachtete ein Indikator dafür ist, dass die Mutante des Pathogens eine Mutation im Virulenzfaktor des Pathogens aufweist, die die Virulenz des Pathogens auf den Wirtsorganismus reduziert.

**8.** Verfahren nach Anspruch 7, worin der einzellige Testorganismus bei dem in Schritt (d) bestimmten Verhältnis und unabhängig voneinander in einer oder mehreren Konzentrationen gegenüber dem Pathogen und der Mutante in den Schritten (e) und (f) exponiert wird und worin das Wachstum des Pathogens und das Wachstum der Mutante, die bei dem in Schritt (d) bestimmten Verhältnis und unabhängig voneinander in einer oder mehreren Konzentrationen des einzelligen Testorganismus exponiert wurden, in Schritt (g) beobachtet wird.

9. Verfahren nach einem der Ansprüche 1 bis 8, worin der einzellige Testorganismus ein phagozytischer Einzeller ist, der gegebenenfalls aus einer phagozytischen Amöbe, einem Geißeltierchen und einem Wimpertierchen ausgewählt ist.

10. Verfahren nach Anspruch 9, worin der einzellige Testorganismus Tetrahymena ist.

11. Verfahren nach Anspruch 10, worin Tetrahymena Tetrahymena pyriformis oder Tetrahymena thermophila ist.

12. Verfahren nach einem der Ansprüche 1 bis 11, worin das Pathogen aus der aus Bakterien, Mycobakterien, Pilzen und einzelligen eukaryotischen Organismen bestehenden Gruppe ausgewählt ist.

13. Verfahren nach Anspruch 12, worin das Pathogen ein extrazelluläres bakterielles Pathogen ist.

14. Verfahren nach Anspruch 13, worin das extrazelluläre bakterielle Pathogen aus der aus Klebsiella pneumoniae, Pseudomonas aeruginosa und Escherichia coli bestehenden Gruppe ausgewählt ist.

15. Verfahren nach einem der Ansprüche 1 bis 14, worin der Wirtsorganismus ein Wirbeltier ist.

## Revendications

1. Procédé d'identification et/ou de quantification de l'activité anti-infectieuse d'un composé anti-infectieux, ledit procédé comprenant:

    (a) exposer un organisme test unicellulaire à un pathogène d'un organisme hôte en l'absence du composé anti-infectieux à identifier et/ou à quantifier;
    (b) déterminer le rapport de la concentration de l'organisme test unicellulaire à celui du pathogène qui permet la croissance du pathogène en présence de l'organisme test unicellulaire;
    (c) exposer l'organisme test unicellulaire au pathogène au rapport déterminé à l'étape (b) en présence du composé anti-infectieux à identifier et/ou à quantifier;
    (d) exposer l'organisme test unicellulaire au pathogène au rapport déterminé à l'étape (b) en l'absence du composé anti-infectieux à identifier et/ou à quantifier; et
    (e) surveiller la croissance du pathogène en présence et en l'absence du composé anti-infectieux à identifier et/ou à quantifier, où un niveau inférieur de croissance du pathogène en présence du composé anti-infectieux à identifier et/ou à quantifier que celui observé en l'absence du composé anti-infectieux à identifier et/ou à quantifier indique que le composé anti-infectieux à identifier et/ou à quantifier possède une activité anti-infectieuse.

2. Procédé selon la revendication 1, dans lequel l'organisme test unicellulaire est exposé à l'étape (c) au rapport déterminé à l'étape (b) et indépendamment à une ou plusieurs concentrations au pathogène en présence du composé anti-infectieux à identifier et/ou à quantifier.

3. Procédé selon la revendication 1 ou la revendication 2, dans lequel simultanément et sous les mêmes conditions de l'étape (c) et (d), le pathogène est exposé sans organisme test unicellulaire en présence et en l'absence du composé anti-infectieux à identifier et/ou à quantifier, et la croissance du pathogène en présence et en l'absence du composé anti-infectieux à identifier et/ou à quantifier est surveillée d'une manière correspondante à l'étape (d), où le niveau de croissance obtenu pour le pathogène en présence et en l'absence du composé anti-infectieux à identifier et/ou à quantifier est utilisé pour calculer l'activité antibiotique du composé anti-infectieux et pour distinguer l'activité antivirulence de l'activité antibiotique du composé anti-infectieux.

4. Procédé selon l'une quelconque des revendications 1 à 3, dans lequel le composé anti-infectieux à identifier et/ou à quantifier est sélectionné parmi

    (1) un composé qui réduit la virulence d'un pathogène à un organisme hôte et l'activité anti-infectieuse du composé est l'activité anti-virulence du composé, et
    (2) un composé antibiotique qui inhibe la croissance ou tue un pathogène en présence et/ou en l'absence d'un organisme hôte, et l'activité anti-infectieuse du composé est l'activité antibiotique du composé.

**5.** Procédé selon l'une quelconque des revendications 1 à 4, dans lequel le procédé est un procédé pour quantifier le $IC_{50}$ d'un composé anti-infectieux, où les étapes (c) à (e) du procédé sont les suivantes:

(c) exposer l'organisme test unicellulaire au pathogène selon le rapport déterminé à l'étape (b) indépendamment en présence de deux ou plusieurs concentrations du composé anti-infectieux;
(d) exposer l'organisme test unicellulaire au pathogène au rapport déterminé à l'étape (b) en l'absence du composé anti-infectieux; et
(e) surveiller la croissance du pathogène indépendamment en présence de deux ou plusieurs concentrations du composé anti-infectieux et en l'absence du composé anti-infectieux, où le niveau de croissance du pathogène en présence de deux ou plusieurs concentrations du composé anti-infectieux et le niveau de croissance du pathogène en l'absence du composé anti-infectieux sont utilisés pour calculer le $IC_{50}$ du composé anti-infectieux.

**6.** Procédé selon la revendication 5, dans lequel l'organisme test unicellulaire est exposé au pathogène à l'étape c) additionnellement, indépendamment en présence d'un composé standard, et la croissance du pathogène est surveillée à l'étape e) indépendamment, en présence de deux ou plusieurs concentrations du composé anti-infectieux, en l'absence du composé anti-infectieux et en présence du composé standard, et où le niveau de croissance du pathogène en présence de deux ou plusieurs concentrations du composé anti-infectieux, le niveau de croissance du pathogène en l'absence du composé anti-infectieux et le niveau de croissance en présence du composé standard sont utilisés pour calculer le $IC_{50}$ du composé anti-infectieux.

**7.** Procédé d'identification et/ou de quantification d'une mutation dans un facteur de virulence d'un pathogène d'un organisme hôte qui réduit la virulence du pathogène à un organisme test unicellulaire, ledit procédé comprenant:

(a) sélectionner un pathogène d'un hôte, dans lequel le pathogène est apte à croître en présence de l'organisme test unicellulaire;
(b) sélectionner un mutant du pathogène;
(c) exposer l'organisme test unicellulaire au pathogène;
(d) déterminer le rapport de la concentration de l'organisme test unicellulaire à celui du pathogène qui permet la croissance du pathogène en présence de l'organisme test unicellulaire;
(e) exposer l'organisme test unicellulaire au pathogène au rapport déterminé à l'étape (d);
(f) exposer l'organisme test unicellulaire au mutant du pathogène au rapport déterminé à l'étape (d); et
(g) surveiller la croissance du pathogène et la croissance du mutant du pathogène, où un niveau plus élevé de croissance du pathogène que celui observé pour le mutant du pathogène indique que le mutant du pathogène possède une mutation dans le facteur de virulence du pathogène qui réduit la virulence du pathogène à l'organisme hôte.

**8.** Procédé selon la revendication 7, dans lequel l'organisme test unicellulaire est exposé au rapport déterminé à l'étape (d) et indépendamment à une ou plusieurs concentrations au pathogène et le mutant aux étapes e) et f), et où la croissance du pathogène et la croissance du mutant exposée au rapport déterminé à l'étape (d) et indépendamment à une ou plusieurs concentrations de l'organisme test unicellulaire est surveillée à l'étape (g).

**9.** Procédé selon l'une quelconque des revendications 1 à 8, dans lequel l'organisme test unicellulaire est un protozoaire phagocytique optionnellement sélectionné parmi une amibe phagocytique, flagellés et ciliés.

**10.** Procédé selon la revendication 9, dans lequel l'organisme test unicellulaire est Tétrahyména.

**11.** Procédé selon la revendication 10, dans lequel Tétrahyména est Tétrahyména pyriformis ou Tétrahyména thermophila.

**12.** Procédé selon l'une quelconque des revendications 1 à 11, dans lequel le pathogène est sélectionné dans le groupe consistant en bactéries, mycobactéries, champignons et organismes eucaryotiques unicellulaires.

**13.** Procédé selon la revendication 12, dans lequel le pathogène est un pathogène bactérien extracellulaire.

**14.** Procédé selon la revendication 13, dans lequel le pathogène bactérien extracellulaire est sélectionné dans le groupe consistant en Klebsiella pneumoniae, Pseudomonas aeruginosa et Escherichia coli.

**15.** Procédé selon l'une quelconque des revendications 1 à 14, dans lequel l'organisme hôte est un vertébré.

Figure 1

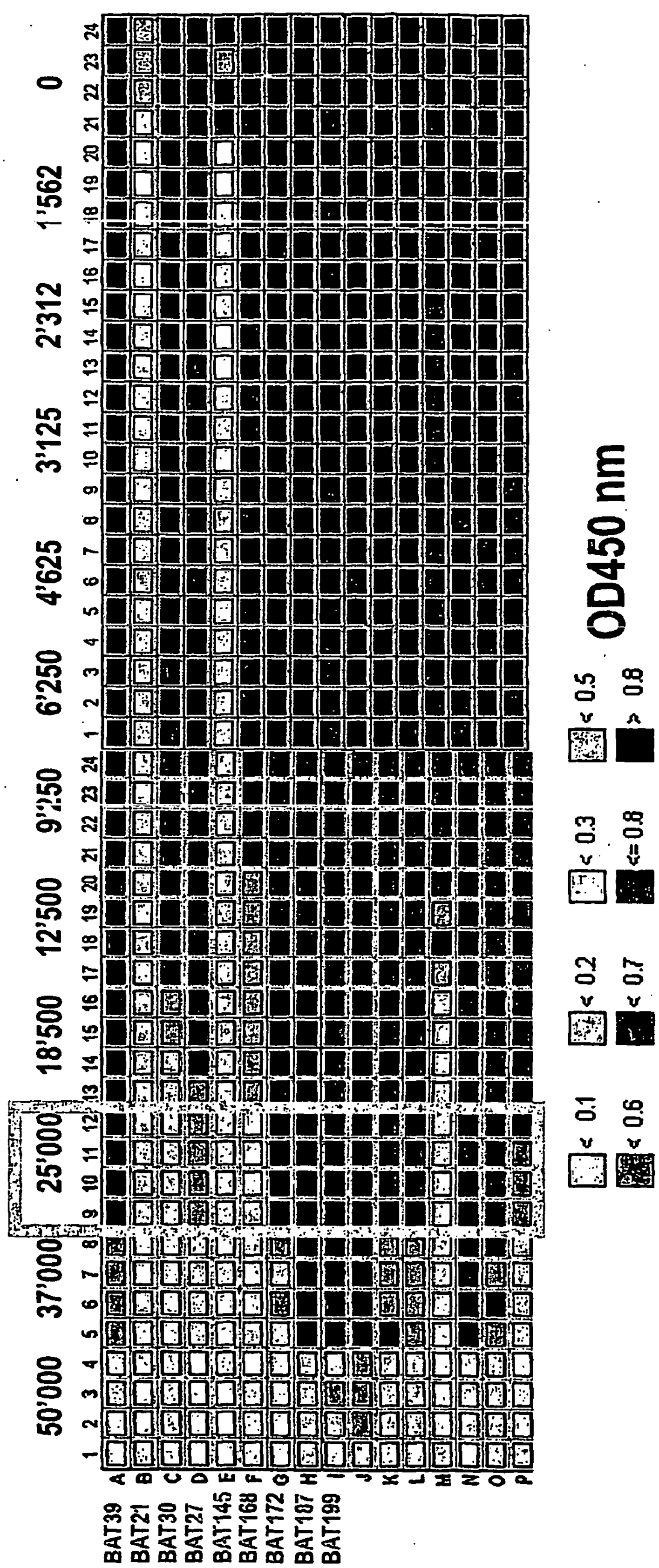
50'000
37'000
25'000
18'500
12'500
9'250
6'250
4'625
3'125
2'312
1'562
0
BAT39 A
BAT21 B
BAT30 C
BAT27 D
BAT145 E
BAT168 F
BAT172 G
BAT107 H
BAT199 I
J
K
L
M
N
O
P
< 0.1
< 0.2
< 0.3
< 0.5
< 0.6
< 0.7
<= 0.8
> 0.8
OD450 nm

**Figure 2**

| | 50'000 | | | | 37'000 | | | | 25'000 | | | |
|---|---|---|---|---|---|---|---|---|---|---|---|---|
| BAT39 | 0.3242 | 0.3318 | 0.3502 | 0.359 | 0.5247 | 0.5209 | 0.5219 | 0.5116 | 0.6852 | 0.6871 | 0.6869 | 0.6453 |
| BAT21 | 0.1749 | 0.2219 | 0.2448 | 0.2051 | 0.224 | 0.2004 | 0.2123 | 0.1644 | 0.1793 | 0.1922 | 0.1652 | 0.1692 |
| BAT30 | 0.1657 | 0.1742 | 0.1732 | 0.1598 | 0.1632 | 0.1581 | 0.1626 | 0.151 | 0.2169 | 0.1803 | 0.1754 | 0.1947 |
| BAT27 | 0.2828 | 0.3135 | 0.3147 | 0.3223 | 0.3843 | 0.4003 | 0.4122 | 0.4195 | 0.5089 | 0.5388 | 0.5031 | 0.5266 |
| BAT145 | 0.2528 | 0.2562 | 0.3098 | 0.1785 | 0.2928 | 0.2795 | 0.2966 | 0.2691 | 0.2317 | 0.2631 | 0.2022 | 0.214 |
| BAT168 | 0.1753 | 0.2855 | 0.388 | 0.2361 | 0.3437 | 0.2945 | 0.2974 | 0.1971 | 0.3438 | 0.2933 | 0.3006 | 0.2814 |
| BAT172 | 0.244 | 0.2804 | 0.2934 | 0.283 | 0.4191 | 0.4557 | 0.3873 | 0.5001 | 0.6215 | 0.6286 | 0.622 | 0.6678 |
| BAT187 | 0.3647 | 0.3976 | 0.4481 | 0.4084 | 0.5969 | 0.6311 | 0.6152 | 0.6459 | 0.7095 | 0.722 | 0.7114 | 0.7451 |
| BAT199 | 0.2864 | 0.4268 | 0.4914 | 0.3559 | 0.6494 | 0.6393 | 0.5723 | 0.6037 | 0.6807 | 0.7116 | 0.6657 | 0.6387 |
| | 0.3258 | 0.4522 | 0.5182 | 0.4917 | 0.6713 | 0.6684 | 0.6172 | 0.6605 | 0.7738 | 0.7636 | 0.7462 | 0.7394 |
| | 0.2857 | 0.3458 | 0.3586 | 0.3896 | 0.572 | 0.5575 | 0.5411 | 0.55 | 0.7195 | 0.7087 | 0.7019 | 0.6916 |
| | 0.293 | 0.3389 | 0.3405 | 0.3444 | 0.5056 | 0.5463 | 0.5364 | 0.5503 | 0.6947 | 0.6773 | 0.6408 | 0.6356 |
| ATH18534 | 0.1687 | 0.2194 | 0.2695 | 0.2735 | 0.2975 | 0.3244 | 0.2498 | 0.259 | 0.2954 | 0.316 | 0.2283 | 0.2187 |
| | 0.2946 | 0.4059 | 0.4349 | 0.4286 | 0.6057 | 0.5783 | 0.603 | 0.6198 | 0.7321 | 0.7602 | 0.7039 | 0.6805 |
| | 0.3079 | 0.3385 | 0.3527 | 0.3925 | 0.5496 | 0.5781 | 0.5149 | 0.5759 | 0.6818 | 0.6859 | 0.6669 | 0.6595 |
| | 0.2887 | 0.2779 | 0.2888 | 0.2849 | 0.4122 | 0.4442 | 0.4356 | 0.4373 | 0.5462 | 0.5565 | 0.5541 | 0.5702 |

| | 18'500 | | | | 12'500 | | | | 9'250 | | | |
|---|---|---|---|---|---|---|---|---|---|---|---|---|
| BAT39 | 0.7139 | 0.7124 | 0.7133 | 0.7361 | 0.7389 | 0.7392 | 0.7429 | 0.7357 | 0.7852 | 0.778 | 0.771 | 0.7448 |
| BAT21 | 0.1384 | 0.1466 | 0.1538 | 0.1361 | 0.1618 | 0.1458 | 0.1408 | 0.1397 | 0.1304 | 0.14 | 0.1397 | 0.1517 |
| BAT30 | 0.358 | 0.435 | 0.4685 | 0.5247 | 0.6252 | 0.6281 | 0.6447 | 0.6749 | 0.7243 | 0.7213 | 0.7283 | 0.6951 |
| BAT27 | 0.5597 | 0.5824 | 0.584 | 0.6105 | 0.6092 | 0.6079 | 0.6059 | 0.6159 | 0.6376 | 0.6365 | 0.6276 | 0.5699 |
| BAT145 | 0.1649 | 0.1785 | 0.1646 | 0.1533 | 0.1615 | 0.1444 | 0.1825 | 0.1305 | 0.1371 | 0.1381 | 0.1434 | 0.2412 |
| BAT168 | 0.4734 | 0.454 | 0.46 | 0.4766 | 0.5408 | 0.5489 | 0.5218 | 0.5324 | 0.5916 | 0.6272 | 0.6175 | 0.5788 |
| BAT172 | 0.7208 | 0.724 | 0.7113 | 0.7669 | 0.7579 | 0.7663 | 0.7583 | 0.7745 | 0.7676 | 0.7837 | 0.784 | 0.7346 |
| BAT187 | 0.775 | 0.7833 | 0.7684 | 0.8021 | 0.8015 | 0.7991 | 0.797 | 0.791 | 1.1528 | 0.8226 | 0.8105 | 0.7777 |
| BAT199 | 0.7274 | 0.7186 | 0.7018 | 0.6893 | 0.724 | 0.7207 | 0.7139 | 0.7087 | 0.7172 | 0.7272 | 0.7268 | 0.6681 |
| | 0.803 | 0.7988 | 0.8118 | 0.8345 | 0.8412 | 0.8298 | 0.8214 | 0.8165 | 0.8346 | 0.8508 | 0.8359 | 0.7984 |
| | 0.7553 | 0.7686 | 0.7468 | 0.758 | 0.7894 | 0.794 | 0.7814 | 0.7804 | 0.7946 | 0.8124 | 0.7968 | 0.7743 |
| | 0.7347 | 0.7598 | 0.7455 | 0.7602 | 0.7869 | 0.782 | 0.7644 | 0.7776 | 0.7825 | 0.7895 | 0.8023 | 0.7569 |
| ATH18534 | 0.1821 | 0.1727 | 0.2137 | 0.2653 | 0.535 | 0.6002 | 0.5232 | 0.5686 | 0.7021 | 0.7096 | 0.7027 | 0.6803 |
| | 0.7987 | 0.8051 | 0.7978 | 0.7936 | 0.8085 | 0.8254 | 0.8028 | 0.8037 | 0.8271 | 0.8306 | 0.8179 | 0.7681 |
| | 0.744 | 0.7472 | 0.7374 | 0.7456 | 0.7724 | 0.7675 | 0.7671 | 0.7592 | 0.7833 | 0.7849 | 0.7774 | 0.732 |
| | 0.6605 | 0.6591 | 0.6865 | 0.6829 | 0.7256 | 0.7208 | 0.7101 | 0.7248 | 0.7418 | 0.7605 | 0.7514 | 0.7364 |

| | 6'250 | | | | 4'625 | | | | 3'125 | | | |
|---|---|---|---|---|---|---|---|---|---|---|---|---|
| BAT39 | 0.8124 | 0.8363 | 0.8166 | 0.8185 | 0.804 | 0.8007 | 0.8265 | 0.8236 | 0.8271 | 0.8398 | 0.8161 | 0.8368 |
| BAT21 | 0.1589 | 0.1408 | 0.1435 | 0.1509 | 0.1245 | 0.1271 | 0.1292 | 0.1284 | 0.1213 | 0.1221 | 0.1276 | 0.1227 |
| BAT30 | 0.7522 | 0.7622 | 0.7718 | 0.7894 | 0.8016 | 0.8066 | 0.8001 | 0.806 | 0.8033 | 0.7501 | 0.7721 | 0.8112 |
| BAT27 | 0.8154 | 0.673 | 0.6719 | 0.682 | 0.6677 | 0.6742 | 0.6656 | 0.6796 | 0.678 | 0.6769 | 0.6553 | 0.6738 |
| BAT145 | 0.1393 | 0.1556 | 0.1333 | 0.1342 | 0.1263 | 0.1354 | 0.153 | 0.1444 | 0.1667 | 0.2213 | 0.1617 | 0.269 |
| BAT168 | 0.6677 | 0.6948 | 0.6996 | 0.6782 | 0.6745 | 0.7128 | 0.7108 | 0.7056 | 0.668 | 0.7305 | 0.7120 | 0.702 |
| BAT172 | 0.7814 | 0.803 | 0.8094 | 0.823 | 0.8226 | 0.8145 | 0.8174 | 0.8393 | 0.8129 | 0.8293 | 0.817 | 0.8306 |
| BAT187 | 0.8024 | 0.8494 | 0.8813 | 0.8686 | 0.8565 | 0.8606 | 0.8648 | 0.88 | 0.862 | 0.873 | 0.8656 | 0.8667 |
| BAT199 | 0.6891 | 0.7511 | 0.7755 | 0.7564 | 0.7826 | 0.765 | 0.7773 | 0.7676 | 0.7624 | 0.7783 | 0.7720 | 0.7683 |
| | 0.8141 | 0.8319 | 0.8757 | 0.881 | 0.8103 | 0.7952 | 0.8043 | 0.8807 | 0.8587 | 0.8357 | 0.8176 | 0.8149 |
| | 0.8179 | 0.8437 | 0.8744 | 0.8684 | 0.8278 | 0.8624 | 0.8228 | 0.825 | 0.8141 | 0.8478 | 0.8538 | 0.819 |
| | 0.7576 | 0.8357 | 0.8211 | 0.8172 | 0.8284 | 0.813 | 0.83 | 0.8157 | 0.8037 | 0.8589 | 0.8287 | 0.8161 |
| ATH18534 | 0.6574 | 0.6352 | 0.7071 | 0.7771 | 0.7421 | 0.7271 | 0.7372 | 0.7508 | 0.737 | 0.7704 | 0.7684 | 0.7256 |
| | 0.73 | 0.7511 | 0.818 | 0.843 | 0.7999 | 0.8496 | 0.8467 | 0.7931 | 0.8404 | 0.8309 | 0.8535 | 0.8077 |
| | 0.7249 | 0.805 | 0.8372 | 0.795 | 0.8084 | 0.7912 | 0.7853 | 0.8085 | 0.8169 | 0.8285 | 0.8373 | 0.825 |
| | 0.7201 | 0.7649 | 0.7683 | 0.7723 | 0.7726 | 0.7704 | 0.7618 | 0.7902 | 0.7883 | 0.8306 | 0.812 | 0.7999 |

| | 2'312 | | | | 1'526 | | | | 0 | | | |
|---|---|---|---|---|---|---|---|---|---|---|---|---|
| BAT39 | 0.8356 | 0.8281 | 0.8192 | 0.8302 | 0.8314 | 0.8118 | 0.821 | 0.8073 | 0.8144 | 0.8289 | 0.8088 | 0.7793 |
| BAT21 | 0.1577 | 0.1163 | 0.1493 | 0.1435 | 0.1389 | 0.1324 | 0.1767 | 0.1882 | 0.3931 | 0.4524 | 0.4632 | 0.5194 |
| BAT30 | 0.7628 | 0.7766 | 0.7718 | 0.7653 | 0.7667 | 0.7902 | 0.7639 | 0.7976 | 0.7624 | 0.7675 | 0.6987 | 0.8229 |
| BAT27 | 0.6559 | 0.6536 | 0.6692 | 0.6847 | 0.6493 | 0.6673 | 0.6519 | 0.6691 | 0.654 | 0.6584 | 0.625 | 0.6209 |
| BAT145 | 0.2865 | 0.2986 | 0.2834 | 0.3791 | 0.3088 | 0.3293 | 0.3228 | 0.3954 | 0.6258 | 0.6265 | 0.529 | 0.7397 |
| BAT168 | 0.6778 | 0.6977 | 0.6879 | 0.7438 | 0.7359 | 0.7632 | 0.7092 | 0.6936 | 0.679 | 0.7581 | 0.7624 | 0.7435 |
| BAT172 | 0.8108 | 0.8208 | 0.8233 | 0.8448 | 0.7942 | 0.8091 | 0.8086 | 0.8159 | 0.8037 | 0.8172 | 0.8085 | 0.8138 |
| BAT187 | 0.8464 | 0.8594 | 0.8258 | 0.8473 | 0.8041 | 0.8548 | 0.844 | 0.8337 | 0.807 | 0.85 | 0.8489 | 0.8326 |
| BAT199 | 0.7605 | 0.7541 | 0.7588 | 0.7646 | 0.7223 | 0.7212 | 0.7146 | 0.7488 | 0.7169 | 0.7407 | 0.7338 | 0.7322 |
| | 0.8655 | 0.8827 | 0.858 | 0.8655 | 0.854 | 0.8518 | 0.8463 | 0.8385 | 0.8181 | 0.8535 | 0.8186 | 0.8105 |
| | 0.8245 | 0.8368 | 0.8527 | 0.8326 | 0.8328 | 0.8428 | 0.8316 | 0.8274 | 0.8332 | 0.8454 | 0.7909 | 0.7765 |
| | 0.8414 | 0.8593 | 0.8689 | 0.8841 | 0.8541 | 0.8642 | 0.8662 | 0.8534 | 0.8582 | 0.859 | 0.8513 | 0.8542 |
| ATH18534 | 0.7038 | 0.7813 | 0.7946 | 0.8051 | 0.7634 | 0.8011 | 0.7895 | 0.7934 | 0.7927 | 0.7514 | 0.7711 | 0.7575 |
| | 0.8374 | 0.8168 | 0.815 | 0.8271 | 0.8376 | 0.8243 | 0.8407 | 0.8024 | 0.8024 | 0.8067 | 0.797 | 0.784 |
| | 0.7907 | 0.8164 | 0.8336 | 0.8298 | 0.7934 | 0.8331 | 0.8251 | 0.801 | 0.825 | 0.8247 | 0.8108 | 0.784 |
| | 0.7897 | 0.8383 | 0.8475 | 0.8686 | 0.8304 | 0.8338 | 0.8312 | 0.8135 | 0.8382 | 0.834 | 0.8422 | 0.7993 |

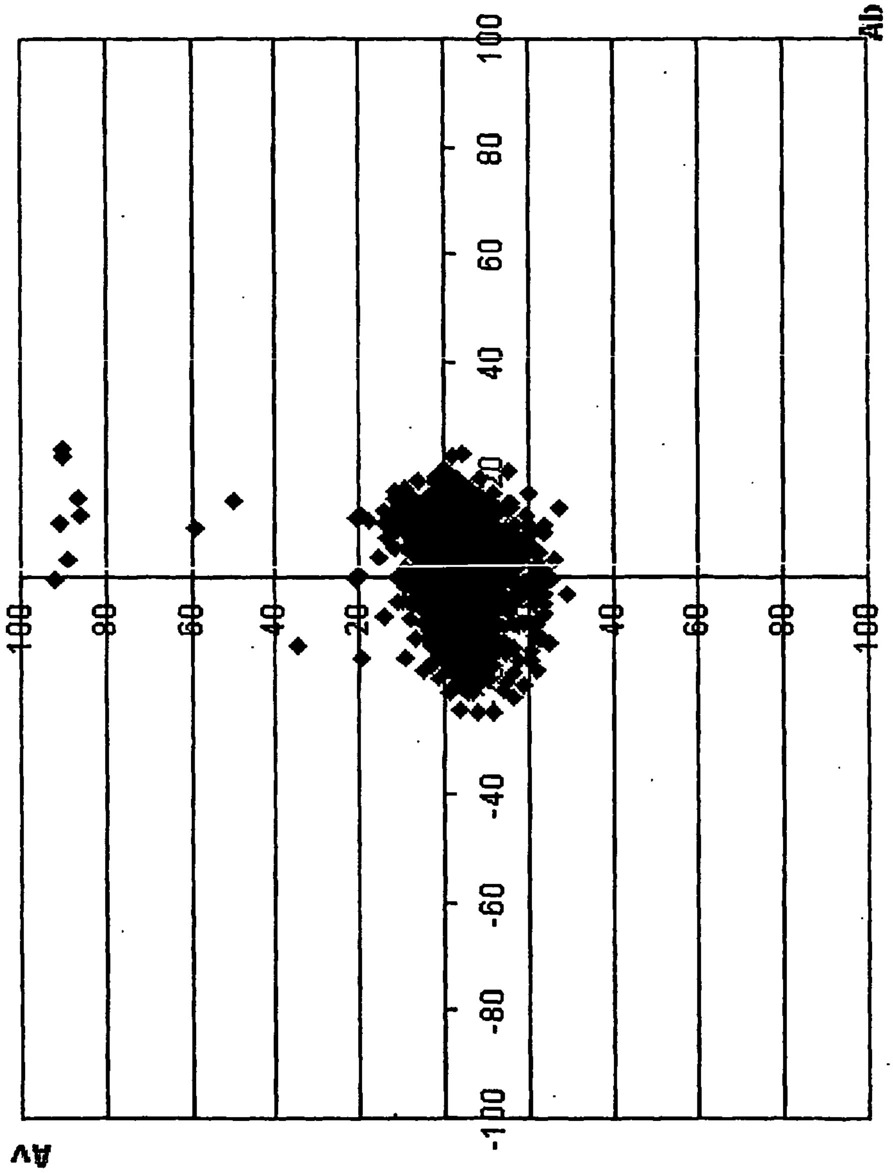


Figure 3

**Figure 4**

| | 50'000 | | | | 37'000 | | | | 25'000 | | | | 18'500 | | | | 12'500 | | | | 9'250 | | | |
|---|---|---|---|---|---|---|---|---|---|---|---|---|---|---|---|---|---|---|---|---|---|---|---|---|
| BAT452 | 0.168 | 0.1884 | 0.2061 | 0.2232 | 0.2175 | 0.285 | 0.2744 | 0.3324 | 0.3187 | 0.2454 | 1.5184 | 0.2779 | 0.2809 | 0.1975 | 0.2912 | 0.3179 | 0.2635 | 0.3446 | 0.2914 | 0.3047 | 0.1733 | 0.2061 | 0.3398 | 0.6548 |
| BAT501 | 0.1831 | 0.1745 | 0.1803 | 0.185 | 0.1677 | 0.1929 | 0.1733 | 0.1905 | 0.1603 | 0.1727 | 0.1688 | 0.1742 | 0.1503 | 0.1521 | 0.1591 | 0.1739 | 0.1693 | 0.1624 | 0.157 | 0.1897 | 0.1509 | 0.1538 | 0.1563 | 0.1709 |
| BAT205 | 0.1735 | 0.1824 | 0.1856 | 0.1846 | 0.1773 | 0.1767 | 0.1704 | 0.1787 | 0.1701 | 0.1703 | 0.1696 | 0.1748 | 0.1614 | 0.1725 | 0.1677 | 0.1766 | 0.171 | 0.1671 | 0.1724 | 0.1734 | 0.1706 | 0.1713 | 0.1746 | 0.1888 |
| BAT503 | 0.1883 | 0.1857 | 0.1848 | 0.2007 | 0.167 | 0.1621 | 0.1715 | 0.1757 | 0.1753 | 0.1729 | 0.1679 | 0.166 | 0.178 | 0.175 | 0.1861 | 0.1859 | 0.1878 | 0.186 | 0.1769 | 0.1775 | 0.1866 | 0.1823 | 0.1839 | 0.1878 |
| BAT505 | 0.1983 | 0.201 | 0.1924 | 0.2075 | 0.1819 | 0.1807 | 0.1918 | 0.2286 | 0.2012 | 0.2216 | 0.195 | 0.1954 | 0.182 | 0.183 | 0.1804 | 0.2083 | 0.1828 | 0.1963 | 0.2012 | 0.191 | 0.1734 | 0.192 | 0.1888 | 0.2068 |
| BAT546 | 0.1788 | 0.1868 | 0.1891 | 0.1829 | 0.1708 | 0.1838 | 0.1848 | 0.1903 | 0.1989 | 0.1903 | 0.2348 | 0.1773 | 0.1762 | 0.2227 | 0.2139 | 0.1927 | 0.1985 | 0.1916 | 0.1893 | 0.1852 | 0.1798 | 0.1822 | 0.1737 | 0.1817 |
| BAT549 | 0.1851 | 0.1643 | 0.1709 | 0.171 | 0.1698 | 0.1714 | 0.1785 | 0.1856 | 0.1732 | 0.1741 | 0.168 | 0.1719 | 0.1649 | 0.1713 | 0.1693 | 0.1781 | 0.1709 | 0.1757 | 0.1628 | 0.163 | 0.154 | 0.1605 | 0.1624 | 0.1677 |
| BAT550 | 0.1848 | 0.1837 | 0.1874 | 0.1949 | 0.1887 | 0.1785 | 0.2044 | 0.1997 | 0.1972 | 0.2048 | 0.2179 | 0.2043 | 0.1928 | 0.1937 | 0.1947 | 0.1908 | 0.7377 | 0.6455 | 0.7086 | 0.1848 | 0.8027 | 0.8485 | 0.8486 | 0.84 |
| BAT563 | 0.1737 | 0.1829 | 0.1829 | 0.186 | 0.1797 | 0.1787 | 0.1898 | 0.1953 | 0.1785 | 0.1857 | 0.1903 | 0.2407 | 0.1903 | 0.186 | 0.1731 | 0.2052 | 0.9477 | 0.1798 | 0.1831 | 0.1975 | 0.9253 | 1.0579 | 1.0371 | 1.0103 |
| | 0.2029 | 0.2583 | 0.1998 | 0.2094 | 0.268 | 0.2761 | 0.2743 | 0.2637 | 0.2818 | 0.2858 | 0.2852 | 0.2705 | 0.2374 | 0.2209 | 0.2271 | 0.2019 | 0.2453 | 0.1991 | 0.2072 | 0.1894 | 0.222 | 0.9637 | 1.0054 | 0.8198 |
| | 0.2372 | 0.2494 | 0.1976 | 0.2106 | 0.2312 | 0.291 | 0.2009 | 0.2827 | 0.2781 | 0.2943 | 0.2908 | 0.2753 | 0.2134 | 0.2405 | 0.2476 | 0.219 | 0.2299 | 0.747 | 0.2087 | 0.2305 | 0.2029 | 0.6019 | 0.9731 | 0.6876 |
| | 0.2211 | 0.2056 | 0.2381 | 0.2413 | 0.2428 | 0.2465 | 0.211 | 0.2859 | 0.2956 | 0.299 | 0.284 | 0.289 | 0.2246 | 0.2776 | 0.2554 | 0.2364 | 0.2232 | 0.2309 | 0.293 | 0.2216 | 0.2152 | 0.5455 | 0.2376 | 0.2486 |
| | 0.1748 | 0.2074 | 0.2114 | 0.2047 | 0.2066 | 0.2639 | 0.2419 | 0.2417 | 0.2704 | 0.266 | 0.2666 | 0.2383 | 0.5232 | 0.6452 | 0.6328 | 0.5752 | 0.2605 | 0.2533 | 0.2377 | 0.204 | 0.204 | 0.6116 | 0.803 | 0.2001 |
| | 0.2051 | 0.2418 | 0.241 | 0.207 | 0.2392 | 0.258 | 0.2501 | 0.2723 | 0.291 | 0.2624 | 0.2495 | 0.2648 | 0.2415 | 0.2596 | 0.2452 | 0.2407 | 0.22 | 0.238 | 0.2052 | 0.207 | 0.2118 | 0.2498 | 0.2019 | 0.2432 |
| | 0.1995 | 0.261 | 0.2408 | 0.2045 | 0.2388 | 0.3082 | 0.2756 | 0.2971 | 0.2712 | 0.2827 | 0.2653 | 0.2755 | 0.266 | 0.2578 | 0.2689 | 0.266 | 0.2259 | 0.2315 | 0.2366 | 0.219 | 0.2045 | 0.2297 | 0.2049 | 0.2465 |
| | 0.1963 | 0.2211 | 0.2648 | 0.2062 | 0.247 | 0.2315 | 0.2272 | 0.2114 | 0.2827 | 0.2689 | 0.2681 | 0.3084 | 0.2992 | 0.2483 | 0.2584 | 0.2617 | 0.2264 | 0.2417 | 0.2188 | 0.2615 | 0.2118 | 0.218 | 0.2236 | 0.2464 |

| | 6'250 | | | | 4'625 | | | | 3'125 | | | | 2'312 | | | | 1'526 | | | | 0 | | | |
|---|---|---|---|---|---|---|---|---|---|---|---|---|---|---|---|---|---|---|---|---|---|---|---|---|
| BAT452 | 0.8412 | 0.4808 | 0.6529 | 0.298 | 1.0376 | 1.0006 | 0.991 | 1.0215 | 1.0754 | 1.0734 | 1.0387 | 1.0567 | 1.0248 | 1.0158 | 1.0084 | 1.0477 | 1.0329 | 1.0343 | 0.9873 | 0.9763 | 0.9587 | 0.9594 | 0.9585 | 1.1526 |
| BAT501 | 0.1537 | 0.1522 | 0.1522 | 0.1612 | 0.144 | 0.1439 | 0.1492 | 0.1609 | 0.1754 | 0.1472 | 0.1471 | 0.1524 | 0.5963 | 0.6347 | 0.3734 | 0.7226 | 0.8121 | 0.8184 | 0.7709 | 0.7573 | 0.8709 | 0.8861 | 0.9039 | 0.9222 |
| BAT205 | 0.1772 | 0.1756 | 0.2901 | 0.1793 | 0.5652 | 0.5616 | 0.4749 | 0.5175 | 0.582 | 0.5626 | 0.5955 | 0.5551 | 0.5761 | 0.5829 | 0.5683 | 0.5947 | 0.6034 | 0.6223 | 0.6067 | 0.5959 | 0.6034 | 0.6142 | 0.6167 | 0.6041 |
| BAT503 | 0.1873 | 0.1834 | 0.1835 | 0.1855 | 0.7603 | 0.7724 | 0.761 | 0.6196 | 0.7995 | 0.8053 | 0.8252 | 0.7986 | 0.8603 | 0.8434 | 0.8584 | 0.8581 | 0.852 | 0.864 | 0.8383 | 0.8432 | 0.8444 | 0.8604 | 0.8477 | 0.8458 |
| BAT505 | 0.9222 | 0.9938 | 0.9078 | 0.9788 | 1.014 | 1.024 | 1.0044 | 1.0635 | 1.0391 | 1.0604 | 1.039 | 1.0777 | 1.0572 | 1.0326 | 1.0355 | 1.0698 | 1.0033 | 1.0425 | 1.0084 | 1.0271 | 0.9664 | 0.9971 | 0.9823 | 1.0298 |
| BAT546 | 0.1661 | 0.1761 | 0.1645 | 0.173 | 0.9261 | 0.8893 | 0.8516 | 0.8746 | 0.9901 | 0.9988 | 0.9629 | 0.9654 | 0.9998 | 1.0116 | 1.0054 | 1.0123 | 0.9794 | 1.0147 | 0.9835 | 0.9682 | 0.975 | 0.9836 | 0.9877 | 0.9643 |
| BAT549 | 0.1599 | 0.1452 | 0.1476 | 0.153 | 0.1489 | 0.1437 | 0.1541 | 0.1561 | 0.152 | 0.1629 | 0.1558 | 0.1576 | 0.165 | 0.403 | 0.2602 | 0.748 | 0.7216 | 0.5865 | 0.6091 | 0.6781 | 0.8691 | 0.8895 | 0.8886 | 0.9085 |
| BAT550 | 0.8776 | 0.8764 | 0.8903 | 0.8923 | 0.9106 | 0.897 | 0.9071 | 0.9207 | 0.9132 | 0.9305 | 0.9041 | 0.9224 | 0.9254 | 0.9348 | 0.9181 | 1.0118 | 0.8877 | 0.9076 | 0.882 | 0.8727 | 0.8564 | 0.8801 | 0.8715 | 0.8586 |
| BAT563 | 1.038 | 1.035 | 1.0398 | 1.0388 | 1.0102 | 1.0488 | 1.0391 | 1.0814 | 1.0611 | 1.0684 | 1.0473 | 1.0612 | 1.0213 | 1.0363 | 1.0642 | 1.1541 | 1.0731 | 1.0597 | 1.033 | 1.0166 | 0.9986 | 1.0144 | 0.998 | 1.0181 |
| | 0.8672 | 0.885 | 0.92 | 0.9104 | 1.0296 | 1.0188 | 1.0027 | 1.0537 | 1.0339 | 1.0883 | 1.0397 | 1.0449 | 1.2983 | 1.0599 | 1.0584 | 1.0835 | 1.0652 | 1.0498 | 1.0228 | 1.0195 | 0.9571 | 0.9802 | 0.9916 | 0.9864 |
| | 0.9411 | 0.8539 | 0.9274 | 1.0012 | 1.0409 | 1.0176 | 1.0252 | 1.0539 | 1.0807 | 1.0764 | 1.0683 | 1.0567 | 1.015 | 1.0489 | 1.0289 | 1.0548 | 1.0717 | 1.0557 | 1.0166 | 1.0099 | 0.9446 | 0.9605 | 0.9429 | 0.956 |
| | 0.8916 | 0.9406 | 0.9298 | 0.9124 | 1.0124 | 1.0291 | 1.0386 | 1.0239 | 1.0813 | 1.0796 | 1.0647 | 1.0587 | 1.018 | 1.0092 | 1.0273 | 1.0308 | 1.0495 | 1.0482 | 1.0201 | 1.0191 | 0.93 | 0.9495 | 0.9498 | 0.9698 |
| | 0.8008 | 0.911 | 0.9074 | 0.9729 | 0.974 | 1.0242 | 1.0048 | 1.0093 | 1.0761 | 1.0719 | 1.0563 | 1.0353 | 1.031 | 1.0293 | 1.0202 | 1.0519 | 1.0165 | 1.0476 | 1.0176 | 1.013 | 0.9727 | 0.9948 | 1.0098 | 0.9961 |
| | 0.902 | 0.9768 | 0.9094 | 0.9882 | 0.9842 | 0.9885 | 0.9939 | 1.0338 | 1.0467 | 1.034 | 1.0423 | 1.0376 | 1.0293 | 1.0239 | 1.0227 | 1.0631 | 1.0514 | 1.0548 | 1.0045 | 1.0182 | 0.9417 | 0.9668 | 0.9565 | 0.9524 |
| | 0.8893 | 0.8861 | 0.9307 | 0.9804 | 1.0284 | 1.012 | 1.0239 | 0.9846 | 1.0513 | 1.0645 | 1.0513 | 1.0393 | 1.007 | 1.0134 | 1.0173 | 1.0375 | 1.0298 | 1.0258 | 1.0091 | 1.0037 | 0.9383 | 0.9589 | 0.9241 | 0.9664 |
| | 0.5923 | 0.856 | 0.4492 | 0.635 | 1.0014 | 0.9973 | 1.006 | 1.0094 | 1.0469 | 1.051 | 1.0521 | 1.0382 | 1.0253 | 1.0402 | 1.0304 | 1.0729 | 1.0402 | 1.0445 | 0.9942 | 1.015 | 0.9558 | 0.9726 | 0.9548 | 0.9625 |

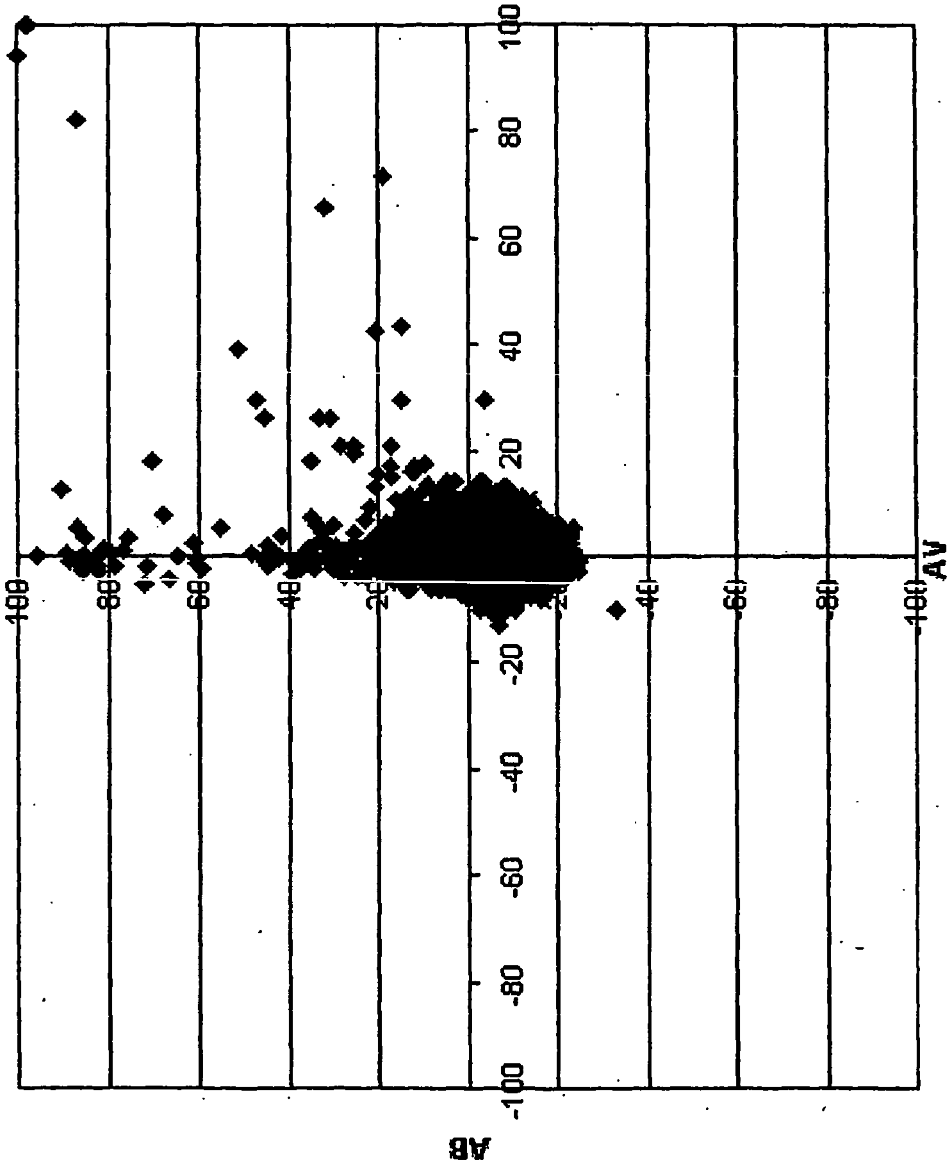


Figure 5

**Figure 6**

| | 50'000 | | | | 37'000 | | | | 25'000 | | | |
|---|---|---|---|---|---|---|---|---|---|---|---|---|
| BAT10 | 1.2912 | 1.4066 | 1.4531 | 1.339 | 1.4088 | 1.4927 | 1.5582 | 1.444 | 1.6542 | 1.5134 | 1.4588 | 1.6363 |
| BAT331 | 0.3903 | 0.1877 | 0.2088 | 0.2476 | 0.188 | 0.2484 | 0.1871 | 0.211 | 0.2072 | 0.2849 | 0.2185 | 1.2115 |
| BAT2 | 0.1602 | 0.2069 | 0.2654 | 0.2937 | 0.1656 | 0.2934 | 0.1658 | 0.2387 | 0.1766 | 0.2105 | 0.2029 | 0.395 |
| BAT334 | 0.1377 | 0.1444 | 0.1606 | 0.1907 | 0.1365 | 0.1469 | 0.1577 | 0.161 | 0.1357 | 0.1446 | 0.1598 | 0.1424 |
| BAT357 | 1.4809 | 0.6009 | 0.361 | 0.559 | 0.6029 | 0.3937 | 0.4668 | 0.8014 | 1.2943 | 1.0893 | 1.1913 | 1.1016 |
| BAT370 | 1.9881 | 1.8893 | 1.9178 | 2.0302 | 1.9592 | 1.9363 | 1.9617 | 2.0752 | 2.0424 | 1.9573 | 1.9674 | 2.0351 |
| BAT375 | 1.3184 | 0.5507 | 1.1816 | 0.8093 | 1.3889 | 1.4862 | 0.4011 | 1.4215 | 1.6245 | 1.3903 | 0.8522 | 1.7975 |
| BAT403 | 0.8874 | 0.4216 | 0.529 | 0.9623 | 1.1738 | 0.4011 | 0.5174 | 0.3316 | 1.3126 | 0.6134 | 0.3216 | 1.3631 |
| BAT408 | 1.5635 | 1.3343 | 1.4209 | 1.4927 | 1.5235 | 1.3417 | 1.3994 | 1.3887 | 1.4837 | 1.3079 | 1.358 | 1.5312 |
| | 1.4663 | 1.238 | 1.4692 | 1.4489 | 1.4894 | 1.5914 | 0.7193 | 1.1468 | 1.6004 | 1.576 | 1.1501 | 1.4814 |
| | 1.5218 | 1.1831 | 1.3608 | 0.4761 | 1.3866 | 1.329 | 1.3219 | 0.5707 | 1.5904 | 1.1752 | 0.9745 | 0.7326 |
| | 1.5131 | 1.2112 | 1.2922 | 0.9389 | 1.4987 | 1.3257 | 1.1224 | 0.7849 | 1.1552 | 1.5058 | 1.4471 | 1.3532 |
| | 1.4702 | 1.4189 | 1.3694 | 1.2516 | 1.0555 | 0.9704 | 1.0486 | 0.842 | 1.3183 | 1.3731 | 1.3419 | 0.8636 |
| | 1.5084 | 1.4049 | 0.8544 | 1.2218 | 1.1157 | 0.8763 | 0.8534 | 0.8055 | 1.355 | 1.1973 | 0.9874 | 0.9323 |
| | 1.4568 | 1.3721 | 0.7227 | 1.2713 | 1.1953 | 1.3387 | 0.8453 | 0.8418 | 1.3614 | 1.3717 | 1.3535 | 0.7142 |
| | 1.1997 | 1.0137 | 1.5238 | 1.3941 | 1.3184 | 1.0586 | 1.3366 | 0.8003 | 1.668 | 1.5829 | 0.6519 | 0.7479 |
| | | | | | | | | | | | | |
| | | | | | | | | | | | | |
| | | | | | | | | | | | | |

| | 18'500 | | | | 12'500 | | | | 9'250 | | | |
|---|---|---|---|---|---|---|---|---|---|---|---|---|
| BAT10 | 1.4687 | 1.3327 | 1.7229 | 1.4076 | 1.4639 | 1.513 | 1.3491 | 1.289 | 1.7171 | 1.6388 | 1.8087 | 1.8571 |
| BAT331 | 0.6971 | 0.6372 | 0.3103 | 0.8034 | 0.6968 | 0.6249 | 0.6641 | 1.2932 | 1.1813 | 1.5172 | 1.5533 | 1.9201 |
| BAT2 | 1.15 | 0.1868 | 0.161 | 0.1693 | 0.1616 | 0.1531 | 0.1747 | 0.317 | 1.0497 | 0.8278 | 0.8301 | 1.772 |
| BAT334 | 0.1322 | 0.1279 | 0.133 | 0.1264 | 0.301 | 0.1338 | 0.1721 | 1.004 | 0.2967 | 0.2246 | 0.2308 | 1.3698 |
| BAT357 | 1.1998 | 0.8702 | 0.6142 | 1.1465 | 1.1162 | 0.9844 | 0.3148 | 1.5543 | 1.1405 | 0.892 | 1.1856 | 1.8779 |
| BAT370 | 2.021 | 1.9752 | 1.9868 | 2.0449 | 2.0122 | 2.0087 | 2.0156 | 2.0432 | 2.0078 | 2.0798 | 2.0651 | 2.01 |
| BAT375 | 1.8053 | 1.5955 | 0.876 | 1.7046 | 1.2787 | 1.2508 | 0.3848 | 1.5834 | 1.6331 | 1.8338 | 1.3916 | 2.0059 |
| BAT403 | 1.4325 | 1.4621 | 1.444 | 1.5127 | 1.5772 | 1.4827 | 1.4693 | 1.6211 | 1.5641 | 1.5449 | 1.6182 | 1.8873 |
| BAT408 | 1.5636 | 1.5812 | 1.5772 | 1.6292 | 1.5961 | 1.5929 | 1.6136 | 1.673 | 1.6478 | 1.7663 | 1.7287 | 1.8741 |
| | 0.1332 | 0.1521 | 0.1588 | 0.101 | 0.1114 | 0.1119 | 0.0953 | 0.0932 | 0.0945 | 0.0888 | 0.0944 | 0.088 |
| | 1.5344 | 1.6809 | 1.4569 | 1.3001 | 1.5185 | 1.5031 | 1.5643 | 1.4908 | 1.4137 | 1.2755 | 1.9034 | 1.743 |
| | 1.5628 | 1.501 | 1.4957 | 1.1972 | 1.2484 | 1.4707 | 1.2966 | 1.3191 | 1.9516 | 1.8098 | 1.8656 | 1.9218 |
| | 1.4951 | 1.559 | 1.3936 | 1.5196 | 1.4517 | 1.5272 | 1.5031 | 1.6003 | 1.5108 | 1.9037 | 1.7951 | 1.825 |
| | 1.5539 | 1.371 | 1.4089 | 1.4418 | 1.2513 | 1.5218 | 1.6101 | 1.59 | 1.8637 | 1.4001 | 1.5681 | 1.8346 |
| | 1.6488 | 1.6545 | 1.5255 | 1.3769 | 1.3682 | 1.6121 | 1.6013 | 1.5473 | 1.6552 | 1.85 | 1.7183 | 1.9172 |
| | 1.4872 | 1.4859 | 1.6778 | 1.7213 | 1.6488 | 1.3771 | 1.8382 | 1.42 | 1.5156 | 1.8157 | 1.7373 | 1.75 |
| | | | | | | | | | | | | |
| | | | | | | | | | | | | |
| | | | | | | | | | | | | |

| | 6'250 | | | | 4'625 | | | | 3'125 | | | |
|---|---|---|---|---|---|---|---|---|---|---|---|---|
| BAT10 | 1.9669 | 1.9553 | 1.8509 | 1.9124 | 1.9912 | 1.9981 | 2.0456 | 2.0335 | 1.9846 | 2.0165 | 2.0104 | 2.0027 |
| BAT331 | 2.0246 | 1.9628 | 1.9626 | 1.7392 | 1.9983 | 2.0105 | 2.0082 | 2.1001 | 2.0094 | 2.0389 | 2.0126 | 2.0256 |
| BAT2 | 1.9305 | 1.8875 | 1.7716 | 1.7207 | 1.9047 | 1.8987 | 1.8968 | 1.983 | 1.9121 | 1.8957 | 1.9054 | 1.9362 |
| BAT334 | 1.4727 | 1.5203 | 1.4619 | 0.7843 | 1.5819 | 1.5889 | 1.5365 | 1.5945 | 1.525 | 1.4355 | 1.4467 | 1.4792 |
| BAT357 | 1.9274 | 1.8643 | 1.8676 | 1.8302 | 2.0242 | 2.0043 | 2.006 | 2.0855 | 1.9986 | 1.9581 | 1.9387 | 2.0843 |
| BAT370 | 2.0244 | 1.9645 | 1.9705 | 1.9098 | 2.0131 | 1.9816 | 1.9561 | 2.0291 | 1.9718 | 1.9719 | 1.9661 | 1.9774 |
| BAT375 | 2.1631 | 2.0264 | 2.1814 | 1.5599 | 2.086 | 2.1745 | 2.1804 | 2.0884 | 2.1587 | 2.1381 | 2.1342 | 2.2197 |
| BAT406 | 1.9831 | 1.8609 | 1.86 | 1.6434 | 1.8761 | 1.7985 | 1.7863 | 1.8118 | 1.8731 | 1.9169 | 1.9202 | 1.8827 |
| BAT408 | 1.6964 | 1.7815 | 1.1783 | 1.1379 | 1.7594 | 1.8065 | 1.7278 | 1.7645 | 1.597 | 1.6719 | 1.6378 | 1.7291 |
| | 2.022 | 1.9932 | 1.8885 | 1.9183 | 2.0191 | 1.9589 | 1.9956 | 1.858 | 2.0182 | 1.9728 | 1.9854 | 2.0094 |
| | 1.9348 | 1.9593 | 1.8834 | 1.8821 | 1.9847 | 1.9802 | 1.9767 | 1.9822 | 2.0283 | 2.0366 | 2.0215 | 2.0324 |
| | 2.0214 | 1.8979 | 1.9248 | 1.9112 | 2.0129 | 2.0166 | 1.9864 | 1.9124 | 2.0282 | 2.0239 | 2.0323 | 2.0277 |
| | 1.9604 | 1.8412 | 1.869 | 1.8605 | 1.986 | 1.9663 | 1.9588 | 1.9305 | 1.9928 | 2.0299 | 2.005 | 1.9811 |
| | 1.9129 | 1.8478 | 1.9449 | 1.9154 | 2.0057 | 2.027 | 1.9447 | 1.9047 | 1.9848 | 2.0272 | 2.0231 | 2.0013 |
| | 2.0301 | 1.9884 | 1.8258 | 1.8491 | 2.0141 | 2.028 | 2.0169 | 1.9408 | 2.0489 | 2.0576 | 2.0371 | 2.0022 |
| | 1.9306 | 2.0263 | 2.039 | 1.8876 | 1.9655 | 1.9255 | 1.9499 | 1.8259 | 2.0542 | 2.0219 | 1.991 | 2.0194 |

| | 2'312 | | | | 1'526 | | | | 0 | | | |
|---|---|---|---|---|---|---|---|---|---|---|---|---|
| BAT10 | 2.033 | 2.0234 | 2.0535 | 2.0601 | 2.0308 | 2.0422 | 2.0368 | 2.0518 | 2.0195 | 2.0336 | 2.0318 | 2.0076 |
| BAT331 | 1.9937 | 1.9517 | 1.9625 | 2.0609 | 2.0143 | 2.02 | 2.0386 | 2.0607 | 2.0263 | 2.0567 | 2.0496 | 2.0466 |
| BAT2 | 1.9085 | 1.8821 | 1.9095 | 1.9604 | 1.8972 | 1.8798 | 1.8761 | 1.9399 | 1.6597 | 1.8509 | 1.8578 | 1.8741 |
| BAT334 | 1.3389 | 1.4273 | 1.3637 | 1.396 | 1.321 | 1.3084 | 1.3348 | 1.3523 | 1.0802 | 1.1113 | 1.1138 | 1.136 |
| BAT357 | 1.9542 | 1.9934 | 1.9917 | 2.0491 | 2.0283 | 1.9826 | 1.9897 | 2.0861 | 1.9877 | 1.9739 | 1.9828 | 2.0848 |
| BAT370 | 1.9949 | 1.976 | 1.9851 | 2.0057 | 1.9832 | 1.9686 | 1.9727 | 1.9879 | 1.9829 | 1.9407 | 1.9885 | 2.0108 |
| BAT375 | 2.1844 | 2.1573 | 2.2004 | 2.2053 | 2.2173 | 2.1789 | 2.2144 | 2.2406 | 2.1883 | 2.1925 | 2.1574 | 2.1447 |
| BAT406 | 1.9958 | 1.9065 | 1.9339 | 1.9118 | 2.0243 | 1.9037 | 1.9566 | 2.02 | 1.0946 | 1.835 | 1.974 | 1.9511 |
| BAT408 | 1.7137 | 1.6307 | 1.6268 | 1.7013 | 1.7042 | 1.679 | 1.6587 | 1.6774 | 1.0154 | 1.8763 | 1.882 | 1.8063 |
| | 2.0226 | 2.0407 | 2.013 | 2.0425 | 2.052 | 2.045 | 2.0504 | 2.0403 | 2.0124 | 1.9988 | 2.0083 | 2.0176 |
| | 2.0621 | 2.0533 | 2.0617 | 2.0603 | 2.0572 | 2.0574 | 2.061 | 2.0498 | 2.036 | 2.0296 | 2.0301 | 2.035 |
| | 2.0511 | 2.0457 | 2.0373 | 2.0539 | 2.0774 | 2.0599 | 2.0617 | 2.0614 | 2.0284 | 2.0365 | 2.042 | 2.04 |
| | 2.0469 | 2.058 | 2.0431 | 2.048 | 2.0571 | 2.0545 | 2.0436 | 2.0519 | 2.0404 | 2.0333 | 2.0383 | 2.032 |
| | 2.0409 | 2.0375 | 2.0625 | 2.0507 | 2.0778 | 2.0665 | 2.0697 | 2.057 | 2.0622 | 2.05 | 2.08 | 2.0265 |
| | 2.0733 | 2.0632 | 2.058 | 2.0605 | 2.0776 | 2.0757 | 2.0775 | 2.0713 | 2.0592 | 2.0661 | 2.0828 | 2.0414 |
| | 2.0375 | 2.0515 | 2.038 | 2.0579 | 2.0871 | 2.0502 | 2.068 | 2.0464 | 2.0364 | 2.0324 | 2.0436 | 1.9838 |

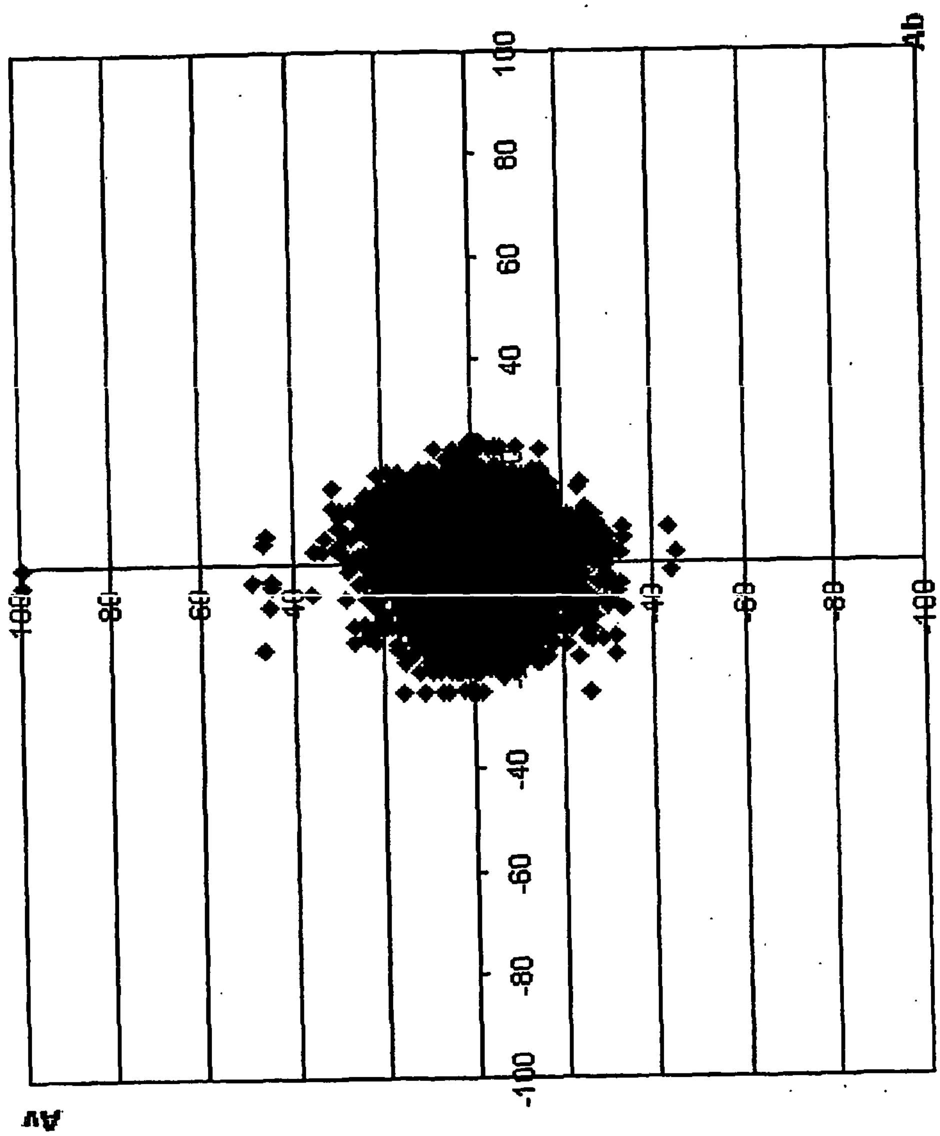


Figure 7

Figure 8

A

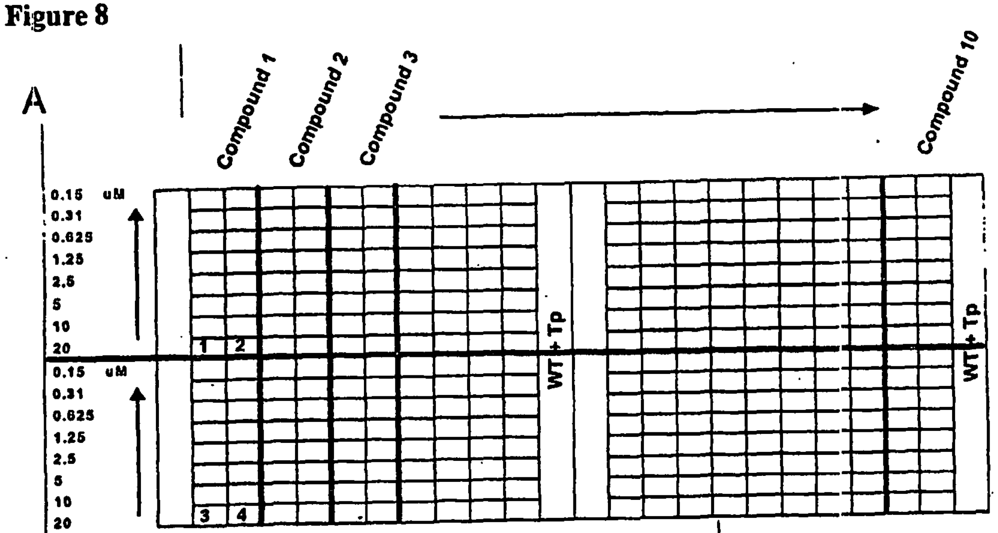


B

| ATH 18534.0505 | 0.15uM | 0.31uM | 0.625uM | 1.25uM | 2.5uM | 5uM | 10nM | 20uM | meanIC50 | sigIC50 |
|---|---|---|---|---|---|---|---|---|---|---|
| Activity | 1.9 | 5.4 | 10.6 | 19.3 | 49.1 | 95.3 | 97.8 | 95.9 | 2.26 | 0.23 |
| OD450 | 0.65 | 0.63 | 0.60 | 0.56 | 0.41 | 0.20 | 0.16 | 0.17 | | |

C

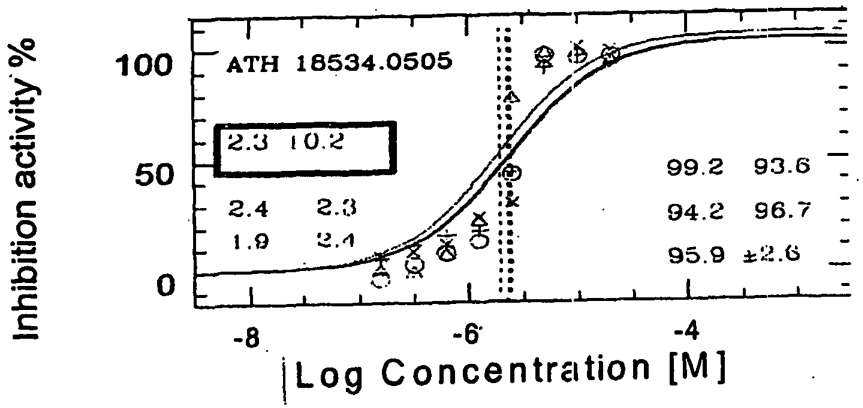

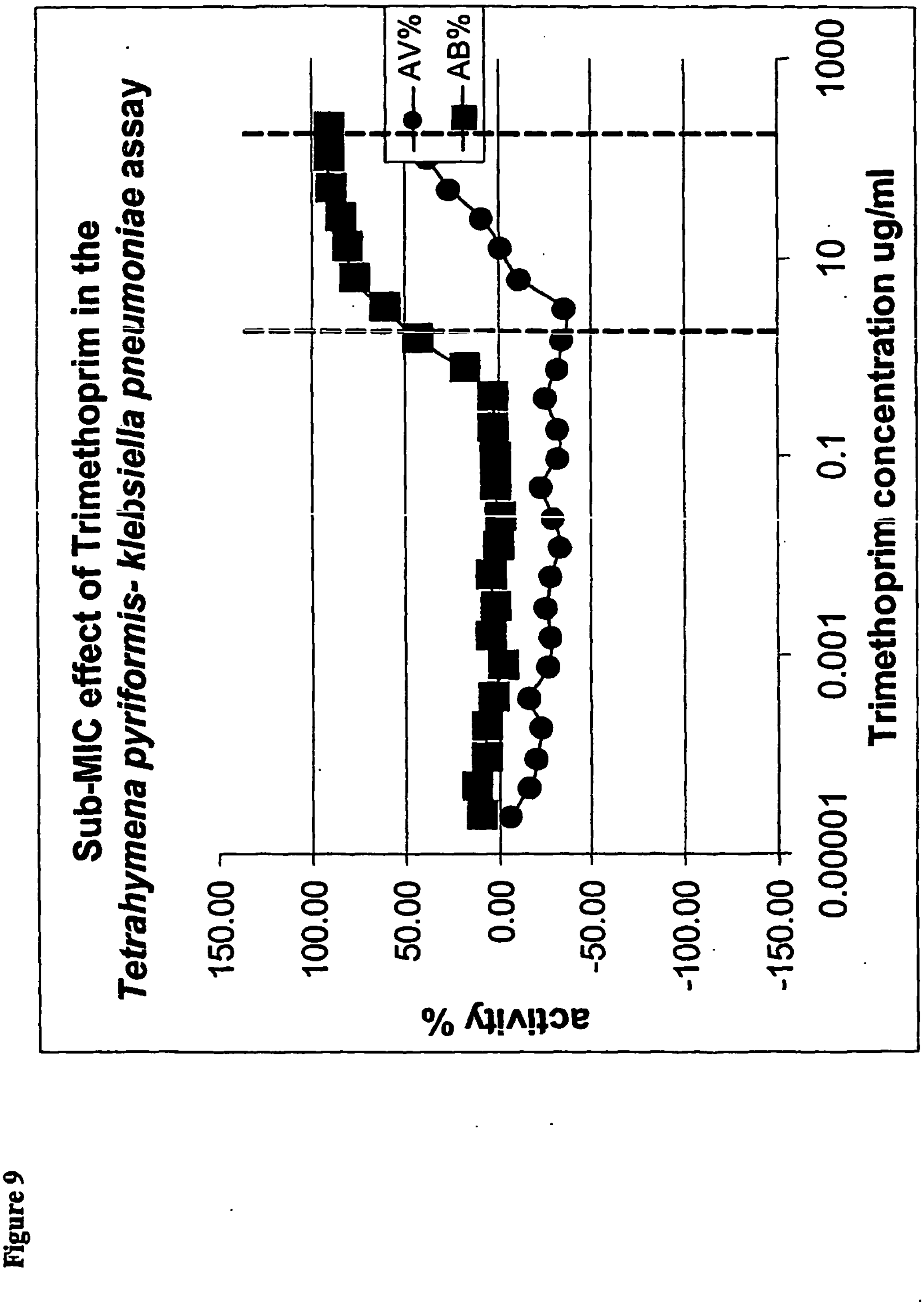

Sub-MIC effect of Trimethoprim in the
*Tetrahymena pyriformis- klebsiella pneumoniae* assay
activity %
150.00
100.00
50.00
0.00
-50.00
-100.00
-150.00
0.00001
0.001
0.1
10
1000
Trimethoprim concentration ug/ml
AV%
AB%


Figure 9

**Figure 10**

| 50'000 | | | | 37'000 | | | | 25'000 | | | | 18'500 | | | | 12'500 | | | | 9'250 | | | |
|---|---|---|---|---|---|---|---|---|---|---|---|---|---|---|---|---|---|---|---|---|---|---|---|
| 0.5722 | 0.5667 | 0.5659 | 0.5566 | 0.6639 | 0.6652 | 0.6519 | 0.6603 | 0.7316 | 0.7504 | 0.7429 | 0.7443 | 0.7483 | 0.7738 | 0.7675 | 0.7786 | 0.8309 | 0.8124 | 0.8342 | 0.8233 | 0.8207 | 0.8339 | 0.8316 | 0.8715 |
| 0.572 | 0.5883 | 0.5977 | 0.6073 | 0.6891 | 0.6817 | 0.6529 | 0.6904 | 0.74 | 0.7389 | 0.721 | 0.7283 | 0.7607 | 0.7636 | 0.7656 | 0.7722 | 0.8427 | 0.8296 | 0.8281 | 0.8371 | 0.8304 | 0.8172 | 0.8327 | 0.8316 |
| 0.5598 | 0.5981 | 0.591 | 0.6065 | 0.6803 | 0.6768 | 0.6461 | 0.6751 | 0.7437 | 0.7283 | 0.7357 | 0.7235 | 0.7542 | 0.743 | 0.7533 | 0.7424 | 0.8025 | 0.7973 | 0.8172 | 0.8234 | 0.8159 | 0.8498 | 0.8263 | 0.841 |
| 0.5782 | 0.5848 | 0.5965 | 0.5895 | 0.6739 | 0.6967 | 0.6862 | 0.6655 | 0.7414 | 0.7762 | 0.7402 | 0.7363 | 0.7601 | 0.7487 | 0.7787 | 0.7812 | 0.8026 | 0.8062 | 0.8016 | 0.8167 | 0.8352 | 0.8289 | 0.853 | 0.8375 |
| 0.5705 | 0.5894 | 0.6089 | 0.585 | 0.7 | 0.6846 | 0.721 | 0.6841 | 0.7639 | 0.7678 | 0.7459 | 0.7579 | 0.7688 | 0.7751 | 0.7829 | 0.7835 | 0.8198 | 0.832 | 0.829 | 0.8247 | 0.8506 | 0.855 | 0.8525 | 0.863 |
| 0.5801 | 0.5768 | 0.5846 | 0.5735 | 0.6865 | 0.6718 | 0.702 | 0.6885 | 0.7589 | 0.769 | 0.7565 | 0.7539 | 0.7854 | 0.7919 | 0.7881 | 0.7941 | 0.8347 | 0.8413 | 0.8407 | 0.8395 | 0.8471 | 0.8639 | 0.8472 | 0.8607 |
| 0.5451 | 0.5813 | 0.5853 | 0.5838 | 0.6827 | 0.6725 | 0.6625 | 0.7 | 0.7616 | 0.7733 | 0.7424 | 0.7644 | 0.7773 | 0.7814 | 0.7806 | 0.8005 | 0.8382 | 0.8163 | 0.815 | 0.8051 | 0.8256 | 0.8441 | 0.8339 | 0.8584 |
| 0.5532 | 0.5909 | 0.5728 | 0.5713 | 0.6784 | 0.6675 | 0.669 | 0.6927 | 0.7516 | 0.7542 | 0.7429 | 0.7605 | 0.78 | 0.7729 | 0.7791 | 0.7876 | 0.8327 | 0.8261 | 0.8181 | 0.8256 | 0.8358 | 0.8529 | 0.8572 | 0.8292 |
| 0.5706 | 0.5889 | 0.608 | 0.5911 | 0.6924 | 0.7146 | 0.6958 | 0.7063 | 0.7586 | 0.7697 | 0.7748 | 0.761 | 0.764 | 0.751 | 0.7781 | 0.7821 | 0.7979 | 0.8163 | 0.8222 | 0.8362 | 0.8232 | 0.8292 | 0.8522 | 0.8353 |
| 0.5701 | 0.6142 | 0.5952 | 0.5908 | 0.7072 | 0.7146 | 0.658 | 0.6948 | 0.7499 | 0.7565 | 0.7561 | 0.7594 | 0.7907 | 0.7795 | 0.7708 | 0.793 | 0.8201 | 0.8185 | 0.8229 | 0.8325 | 0.8133 | 0.8556 | 0.8722 | 0.8271 |
| 0.59 | 0.5932 | 0.6145 | 0.5908 | 0.6872 | 0.7167 | 0.706 | 0.7199 | 0.7609 | 0.7546 | 0.7587 | 0.75 | 0.7909 | 0.7778 | 0.7757 | 0.7843 | 0.8205 | 0.823 | 0.8085 | 0.8335 | 0.8117 | 0.8382 | 0.8456 | 0.8447 |
| 0.5876 | 0.5995 | 0.5912 | 0.6067 | 0.6813 | 0.6927 | 0.6963 | 0.6825 | 0.7707 | 0.7734 | 0.7501 | 0.7779 | 0.785 | 0.7689 | 0.7892 | 0.78 | 0.8064 | 0.8024 | 0.8131 | 0.8333 | 0.8358 | 0.8493 | 0.8532 | 0.8517 |
| 0.5873 | 0.5962 | 0.6061 | 0.6014 | 0.7003 | 0.676 | 0.7022 | 0.7121 | 0.7565 | 0.7586 | 0.7507 | 0.7604 | 0.7791 | 0.7687 | 0.771 | 0.7809 | 0.8207 | 0.7961 | 0.8152 | 0.8372 | 0.8418 | 0.838 | 0.8399 | 0.8398 |
| 0.5635 | 0.6123 | 0.6003 | 0.6024 | 0.6903 | 0.6828 | 0.6895 | 0.6803 | 0.7724 | 0.7495 | 0.7589 | 0.7582 | 0.7825 | 0.7792 | 0.7797 | 0.7849 | 0.8103 | 0.816 | 0.8042 | 0.8252 | 0.8455 | 0.8388 | 0.8394 | 0.839 |
| 0.5804 | 0.607 | 0.5959 | 0.5863 | 0.6743 | 0.6803 | 0.6971 | 0.6598 | 0.7358 | 0.7255 | 0.7372 | 0.7278 | 0.7619 | 0.769 | 0.7671 | 0.7721 | 0.802 | 0.7762 | 0.787 | 0.8056 | 0.8091 | 0.8259 | 0.8121 | 0.8284 |
| 0.5598 | 0.5824 | 0.5795 | 0.5729 | 0.6498 | 0.6501 | 0.6612 | 0.6583 | 0.736 | 0.7303 | 0.7078 | 0.7183 | 0.7624 | 0.7872 | 0.7571 | 0.7826 | 0.815 | 0.7921 | 0.8227 | 0.8252 | 0.8447 | 0.8723 | 0.8666 | 0.8596 |

| 6'250 | | | | 4'625 | | | | 3'125 | | | | 2'312 | | | | 1'526 | | | | 0 | | | |
|---|---|---|---|---|---|---|---|---|---|---|---|---|---|---|---|---|---|---|---|---|---|---|---|
| 0.8254 | 0.8042 | 0.774 | 0.7982 | 0.7858 | 0.8127 | 0.7904 | 0.7985 | 0.831 | 0.8004 | 0.8262 | 0.8049 | 0.8411 | 0.8077 | 0.8218 | 0.8022 | 0.8403 | 0.8381 | 0.8162 | 0.8402 | 0.7766 | 0.7337 | 0.8194 | 0.8466 |
| 0.8 | 0.8014 | 0.7877 | 0.8088 | 0.8051 | 0.8108 | 0.7754 | 0.809 | 0.8173 | 0.7907 | 0.793 | 0.7853 | 0.8332 | 0.822 | 0.7832 | 0.7909 | 0.8361 | 0.8299 | 0.8231 | 0.8435 | 0.8339 | 0.7369 | 0.8395 | 0.8366 |
| 0.7971 | 0.7999 | 0.7649 | 0.8115 | 0.8207 | 0.8239 | 0.7794 | 0.7677 | 0.8256 | 0.8283 | 0.8316 | 0.8105 | 0.7834 | 0.8067 | 0.7979 | 0.8086 | 0.8271 | 0.8315 | 0.8173 | 0.8276 | 0.7216 | 0.7241 | 0.7527 | 0.8333 |
| 0.8444 | 0.7984 | 0.8084 | 0.8256 | 0.8306 | 0.8032 | 0.8131 | 0.7942 | 0.8131 | 0.8413 | 0.815 | 0.828 | 0.7938 | 0.8231 | 0.8121 | 0.8172 | 0.8298 | 0.7958 | 0.8407 | 0.8361 | 0.723 | 0.7233 | 0.7442 | 0.7889 |
| 0.823 | 0.817 | 0.8255 | 0.8192 | 0.8193 | 0.8098 | 0.7884 | 0.7872 | 0.8179 | 0.8372 | 0.8155 | 0.8177 | 0.8319 | 0.8085 | 0.8178 | 0.808 | 0.7937 | 0.8087 | 0.8142 | 0.8373 | 0.7526 | 0.7834 | 0.7954 | 0.8543 |
| 0.8309 | 0.8048 | 0.8034 | 0.8276 | 0.8318 | 0.804 | 0.799 | 0.8368 | 0.8195 | 0.8268 | 0.8064 | 0.8153 | 0.8191 | 0.8166 | 0.8475 | 0.8183 | 0.8178 | 0.8223 | 0.7945 | 0.7987 | 0.7685 | 0.7875 | 0.8333 | 0.8586 |
| 0.8298 | 0.8145 | 0.8175 | 0.8415 | 0.8292 | 0.8059 | 0.8071 | 0.813 | 0.8269 | 0.8253 | 0.8138 | 0.8246 | 0.7851 | 0.8271 | 0.7977 | 0.8302 | 0.8229 | 0.8099 | 0.8045 | 0.8221 | 0.7199 | 0.7409 | 0.741 | 0.8585 |
| 0.8221 | 0.8061 | 0.8143 | 0.821 | 0.8008 | 0.7886 | 0.7624 | 0.7807 | 0.8404 | 0.8289 | 0.8221 | 0.8379 | 0.7917 | 0.818 | 0.7747 | 0.7898 | 0.8303 | 0.8143 | 0.8352 | 0.8216 | 0.8152 | 0.691 | 0.7858 | 0.8239 |
| 0.8207 | 0.8085 | 0.8232 | 0.7944 | 0.8168 | 0.8251 | 0.7946 | 0.7803 | 0.7817 | 0.8218 | 0.7928 | 0.8138 | 0.7889 | 0.7845 | 0.8078 | 0.833 | 0.8283 | 0.8199 | 0.8167 | 0.8405 | 0.7619 | 0.7687 | 0.7465 | 0.7755 |
| 0.8236 | 0.7895 | 0.8156 | 0.8337 | 0.7997 | 0.8292 | 0.7893 | 0.8093 | 0.8343 | 0.826 | 0.8256 | 0.8175 | 0.8418 | 0.8114 | 0.8095 | 0.8369 | 0.8086 | 0.7873 | 0.82 | 0.8092 | 0.7435 | 0.7136 | 0.7327 | 0.795 |
| 0.8261 | 0.8098 | 0.8268 | 0.8181 | 0.8388 | 0.8361 | 0.8065 | 0.8192 | 0.8384 | 0.8438 | 0.8187 | 0.8269 | 0.7959 | 0.8268 | 0.8266 | 0.8136 | 0.8046 | 0.8262 | 0.7955 | 0.8234 | 0.7025 | 0.7256 | 0.738 | 0.8506 |
| 0.848 | 0.8269 | 0.8423 | 0.8323 | 0.852 | 0.8402 | 0.8164 | 0.8466 | 0.839 | 0.8334 | 0.8332 | 0.8364 | 0.8389 | 0.8317 | 0.8309 | 0.8211 | 0.8115 | 0.8334 | 0.818 | 0.8072 | 0.7757 | 0.717 | 0.8198 | 0.8501 |
| 0.8446 | 0.8039 | 0.8218 | 0.8316 | 0.8147 | 0.8419 | 0.7794 | 0.8145 | 0.8341 | 0.7985 | 0.8047 | 0.7922 | 0.789 | 0.8135 | 0.817 | 0.7908 | 0.7615 | 0.7835 | 0.7917 | 0.7995 | 0.7378 | 0.6833 | 0.649 | 0.8408 |
| 0.8292 | 0.8049 | 0.8251 | 0.8166 | 0.8145 | 0.8163 | 0.8129 | 0.8213 | 0.8054 | 0.8262 | 0.82 | 0.7894 | 0.7857 | 0.793 | 0.8205 | 0.8325 | 0.7809 | 0.7919 | 0.8156 | 0.7973 | 0.7363 | 0.8198 | 0.7841 | 0.8286 |
| 0.8202 | 0.8247 | 0.8236 | 0.8322 | 0.8105 | 0.8273 | 0.823 | 0.8292 | 0.8216 | 0.8073 | 0.8084 | 0.8093 | 0.8069 | 0.8043 | 0.8096 | 0.8182 | 0.8147 | 0.8134 | 0.8098 | 0.805 | 0.721 | 0.7512 | 0.693 | 0.7926 |
| 0.8225 | 0.8179 | 0.8367 | 0.8193 | 0.826 | 0.829 | 0.8215 | 0.8198 | 0.8327 | 0.8653 | 0.8267 | 0.8193 | 0.8211 | 0.8572 | 0.8319 | 0.8437 | 0.853 | 0.84 | 0.8428 | 0.8381 | 0.8076 | 0.8649 | 0.8424 | 0.8548 |

Figure 11

| 50'000 | | | | 37'000 | | | | 25'000 | | | | 18'500 | | | | 12'500 | | | | 9'250 | | | |
|---|---|---|---|---|---|---|---|---|---|---|---|---|---|---|---|---|---|---|---|---|---|---|---|
| 0.4198 | 0.4291 | 0.4266 | 0.4334 | 0.5411 | 0.5432 | 0.5614 | 0.5588 | 0.6406 | 0.6438 | 0.6394 | 0.6519 | 0.6859 | 0.6929 | 0.6809 | 0.682 | 0.7029 | 0.716 | 0.7098 | 0.7173 | 0.7163 | 0.7116 | 0.7075 | 0.6804 |
| 0.4164 | 0.4282 | 0.441 | 0.4368 | 0.5574 | 0.5349 | 0.5568 | 0.5636 | 0.648 | 0.6579 | 0.6545 | 0.6455 | 0.7097 | 0.7074 | 0.7247 | 0.716 | 0.7252 | 0.7387 | 0.7266 | 0.7332 | 0.7314 | 0.7388 | 0.728 | 0.7191 |
| 0.4134 | 0.426 | 0.4354 | 0.4595 | 0.5478 | 0.5533 | 0.5448 | 0.5712 | 0.6355 | 0.654 | 0.6429 | 0.6543 | 0.6997 | 0.6935 | 0.71 | 0.7006 | 0.7211 | 0.7342 | 0.7066 | 0.7343 | 0.7437 | 0.7327 | 0.7328 | 0.724 |
| 0.4176 | 0.4392 | 0.4364 | 0.423 | 0.5629 | 0.5665 | 0.5928 | 0.5791 | 0.6753 | 0.6712 | 0.6675 | 0.6792 | 0.7188 | 0.6965 | 0.7092 | 0.7159 | 0.739 | 0.7079 | 0.7212 | 0.7194 | 0.751 | 0.7483 | 0.7531 | 0.7311 |
| 0.4198 | 0.4687 | 0.4515 | 0.4705 | 0.5813 | 0.5848 | 0.5948 | 0.5971 | 0.6914 | 0.6958 | 0.6881 | 0.6832 | 0.7241 | 0.7299 | 0.7485 | 0.7285 | 0.7558 | 0.751 | 0.7434 | 0.7573 | 0.7638 | 0.7645 | 0.7786 | 0.7514 |
| 0.4164 | 0.4181 | 0.4318 | 0.4245 | 0.5737 | 0.5886 | 0.5877 | 0.6031 | 0.6798 | 0.7176 | 0.694 | 0.6927 | 0.7399 | 0.7282 | 0.7422 | 0.7311 | 0.757 | 0.7676 | 0.7465 | 0.7515 | 0.7635 | 0.7727 | 0.7673 | 0.7607 |
| 0.3892 | 0.4123 | 0.4201 | 0.4247 | 0.5716 | 0.5794 | 0.5594 | 0.5804 | 0.6725 | 0.6744 | 0.6875 | 0.6783 | 0.7314 | 0.7121 | 0.7258 | 0.7304 | 0.7541 | 0.7316 | 0.7486 | 0.7341 | 0.7741 | 0.7724 | 0.7687 | 0.7305 |
| 0.4095 | 0.4265 | 0.4209 | 0.4334 | 0.5813 | 0.5696 | 0.5637 | 0.5847 | 0.6794 | 0.6763 | 0.6886 | 0.667 | 0.7429 | 0.7196 | 0.7271 | 0.7264 | 0.7466 | 0.7431 | 0.7605 | 0.7514 | 0.7815 | 0.7708 | 0.775 | 0.7346 |
| 0.4025 | 0.4456 | 0.4387 | 0.442 | 0.578 | 0.5943 | 0.5936 | 0.6022 | 0.68 | 0.7001 | 0.7023 | 0.6922 | 0.73 | 0.7302 | 0.743 | 0.7344 | 0.7594 | 0.7664 | 0.7724 | 0.7604 | 0.7819 | 0.7867 | 0.7305 | 0.7442 |
| 0.4138 | 0.418 | 0.4223 | 0.4611 | 0.5706 | 0.5892 | 0.5966 | 0.5887 | 0.7041 | 0.6772 | 0.6886 | 0.6942 | 0.7272 | 0.7273 | 0.749 | 0.744 | 0.7429 | 0.751 | 0.7571 | 0.758 | 0.8036 | 0.7813 | 0.7502 | 0.7489 |
| 0.4082 | 0.4193 | 0.4399 | 0.4349 | 0.5712 | 0.5637 | 0.5872 | 0.5788 | 0.6807 | 0.6881 | 0.7057 | 0.6888 | 0.7122 | 0.7207 | 0.7223 | 0.7177 | 0.7288 | 0.7398 | 0.7417 | 0.7402 | 0.7631 | 0.7628 | 0.748 | 0.7309 |
| 0.4039 | 0.4384 | 0.4152 | 0.467 | 0.5793 | 0.5924 | 0.5917 | 0.5888 | 0.6897 | 0.6878 | 0.6937 | 0.6843 | 0.7193 | 0.7133 | 0.7272 | 0.718 | 0.7432 | 0.7375 | 0.7369 | 0.7445 | 0.7759 | 0.7687 | 0.7493 | 0.7526 |
| 0.4024 | 0.437 | 0.4252 | 0.4282 | 0.5901 | 0.5498 | 0.6021 | 0.5959 | 0.6972 | 0.6944 | 0.6919 | 0.683 | 0.7192 | 0.7277 | 0.729 | 0.7238 | 0.7561 | 0.7438 | 0.7425 | 0.7549 | 0.7682 | 0.7715 | 0.7428 | 0.7173 |
| 0.4179 | 0.4376 | 0.4088 | 0.4262 | 0.5451 | 0.5528 | 0.5635 | 0.5509 | 0.6593 | 0.6598 | 0.6523 | 0.6637 | 0.7008 | 0.7093 | 0.7059 | 0.7183 | 0.7328 | 0.734 | 0.7377 | 0.7323 | 0.7534 | 0.7505 | 0.7299 | 0.7038 |
| 0.4156 | 0.4303 | 0.4344 | 0.4139 | 0.5408 | 0.5398 | 0.5331 | 0.5417 | 0.6438 | 0.6432 | 0.6363 | 0.639 | 0.6913 | 0.6852 | 0.698 | 0.6812 | 0.7174 | 0.7258 | 0.7183 | 0.7221 | 0.7439 | 0.7222 | 0.7309 | 0.7231 |
| 0.4262 | 0.4451 | 0.4285 | 0.4414 | 0.5536 | 0.5357 | 0.537 | 0.5364 | 0.6272 | 0.6352 | 0.6211 | 0.6322 | 0.6699 | 0.6817 | 0.688 | 0.6611 | 0.7012 | 0.6943 | 0.6857 | 0.7159 | 0.7386 | 0.7152 | 0.7487 | 0.711 |

| 6'250 | | | | 4'625 | | | | 3'125 | | | | 2'312 | | | | 1'526 | | | | 0 | | | |
|---|---|---|---|---|---|---|---|---|---|---|---|---|---|---|---|---|---|---|---|---|---|---|---|
| 0.7023 | 0.721 | 0.7546 | 0.7317 | 0.7118 | 0.702 | 0.7263 | 0.7231 | 0.7014 | 0.7098 | 0.7345 | 0.7212 | 0.7136 | 0.7342 | 0.7264 | 0.7065 | 0.7259 | 0.7312 | 0.704 | 0.7277 | 0.8483 | 0.9051 | 0.8267 | 0.8356 |
| 0.7371 | 0.7428 | 0.7473 | 0.7495 | 0.7398 | 0.7208 | 0.725 | 0.7425 | 0.7314 | 0.716 | 0.7312 | 0.6967 | 0.7183 | 0.7179 | 0.7349 | 0.7347 | 0.7229 | 0.7369 | 0.7224 | 0.7187 | 0.7907 | 0.8112 | 0.8245 | 0.8477 |
| 0.7128 | 0.7536 | 0.7365 | 0.7363 | 0.7254 | 0.7031 | 0.7285 | 0.7306 | 0.7221 | 0.7144 | 0.7287 | 0.7284 | 0.7205 | 0.7282 | 0.7356 | 0.7254 | 0.7241 | 0.7186 | 0.7122 | 0.7011 | 0.7347 | 0.7404 | 0.6976 | 0.7666 |
| 0.7347 | 0.7373 | 0.7383 | 0.737 | 0.7331 | 0.727 | 0.7288 | 0.7226 | 0.7487 | 0.7572 | 0.7433 | 0.7434 | 0.7363 | 0.7212 | 0.7346 | 0.7232 | 0.7334 | 0.7363 | 0.727 | 0.7099 | 0.7294 | 0.7092 | 0.8013 | 0.8492 |
| 0.7525 | 0.7365 | 0.7758 | 0.7603 | 0.7377 | 0.7524 | 0.7514 | 0.7504 | 0.7457 | 0.7394 | 0.7248 | 0.755 | 0.7353 | 0.7467 | 0.7813 | 0.7467 | 0.7678 | 0.7538 | 0.7265 | 0.7311 | 0.8235 | 0.8227 | 0.8363 | 0.8401 |
| 0.7349 | 0.7507 | 0.7581 | 0.739 | 0.7612 | 0.7387 | 0.759 | 0.7699 | 0.7515 | 0.7697 | 0.7594 | 0.7517 | 0.7593 | 0.7629 | 0.7769 | 0.7708 | 0.7398 | 0.7385 | 0.7454 | 0.7515 | 0.8249 | 0.7498 | 0.8302 | 0.8517 |
| 0.741 | 0.7555 | 0.7667 | 0.7465 | 0.7543 | 0.7473 | 0.7497 | 0.7627 | 0.7461 | 0.743 | 0.7634 | 0.7563 | 0.7541 | 0.772 | 0.7721 | 0.7584 | 0.7614 | 0.7469 | 0.7453 | 0.7171 | 0.7441 | 0.7993 | 0.7422 | 0.8179 |
| 0.7314 | 0.7591 | 0.7527 | 0.7408 | 0.7361 | 0.7302 | 0.7587 | 0.7537 | 0.7551 | 0.7583 | 0.767 | 0.7564 | 0.7733 | 0.7624 | 0.7661 | 0.7477 | 0.7677 | 0.7351 | 0.7319 | 0.7189 | 0.7935 | 0.8061 | 0.8027 | 0.7671 |
| 0.7498 | 0.7556 | 0.7849 | 0.7601 | 0.7632 | 0.7646 | 0.7837 | 0.7697 | 0.7623 | 0.757 | 0.7605 | 0.7762 | 0.768 | 0.7755 | 0.7915 | 0.7782 | 0.7601 | 0.7626 | 0.7236 | 0.758 | 0.7537 | 0.8051 | 0.7225 | 0.8643 |
| 0.7241 | 0.7414 | 0.7733 | 0.7561 | 0.7542 | 0.7842 | 0.7589 | 0.7682 | 0.7466 | 0.7682 | 0.7622 | 0.7762 | 0.7474 | 0.7796 | 0.7861 | 0.7848 | 0.7541 | 0.764 | 0.7533 | 0.7288 | 0.798 | 0.7528 | 0.781 | 0.8399 |
| 0.7218 | 0.7489 | 0.7871 | 0.7568 | 0.7503 | 0.7575 | 0.7744 | 0.7611 | 0.748 | 0.7429 | 0.7649 | 0.7751 | 0.7645 | 0.7746 | 0.7758 | 0.7713 | 0.7488 | 0.7568 | 0.753 | 0.7671 | 0.7945 | 0.8047 | 0.7672 | 0.7988 |
| 0.7257 | 0.7528 | 0.7745 | 0.7604 | 0.7516 | 0.7647 | 0.7609 | 0.7471 | 0.7561 | 0.7625 | 0.7527 | 0.7363 | 0.7664 | 0.7731 | 0.7759 | 0.7695 | 0.739 | 0.7585 | 0.7419 | 0.7537 | 0.8259 | 0.81 | 0.8206 | 0.8336 |
| 0.7327 | 0.7559 | 0.7742 | 0.736 | 0.7529 | 0.7521 | 0.7726 | 0.7518 | 0.7516 | 0.7417 | 0.7692 | 0.7684 | 0.7385 | 0.7366 | 0.7573 | 0.7545 | 0.7617 | 0.7424 | 0.7344 | 0.7034 | 0.7944 | 0.7179 | 0.8053 | 0.8143 |
| 0.7154 | 0.7221 | 0.7638 | 0.7356 | 0.7331 | 0.7309 | 0.7403 | 0.7302 | 0.7206 | 0.7454 | 0.7426 | 0.7565 | 0.7338 | 0.738 | 0.7438 | 0.7361 | 0.7338 | 0.7585 | 0.7164 | 0.7201 | 0.7813 | 0.7553 | 0.7678 | 0.8146 |
| 0.7051 | 0.7498 | 0.739 | 0.7438 | 0.7337 | 0.7274 | 0.7215 | 0.7284 | 0.7231 | 0.7239 | 0.7274 | 0.7386 | 0.7307 | 0.7464 | 0.724 | 0.7321 | 0.7256 | 0.7438 | 0.7322 | 0.7232 | 0.7099 | 0.719 | 0.8216 | 0.8496 |
| 0.7211 | 0.7304 | 0.7223 | 0.7026 | 0.7259 | 0.7293 | 0.7207 | 0.6993 | 0.7348 | 0.7186 | 0.7309 | 0.744 | 0.7075 | 0.73 | 0.7301 | 0.7283 | 0.7401 | 0.7453 | 0.7314 | 0.7081 | 0.8258 | 0.7655 | 0.8324 | 0.8653 |

Figure 12

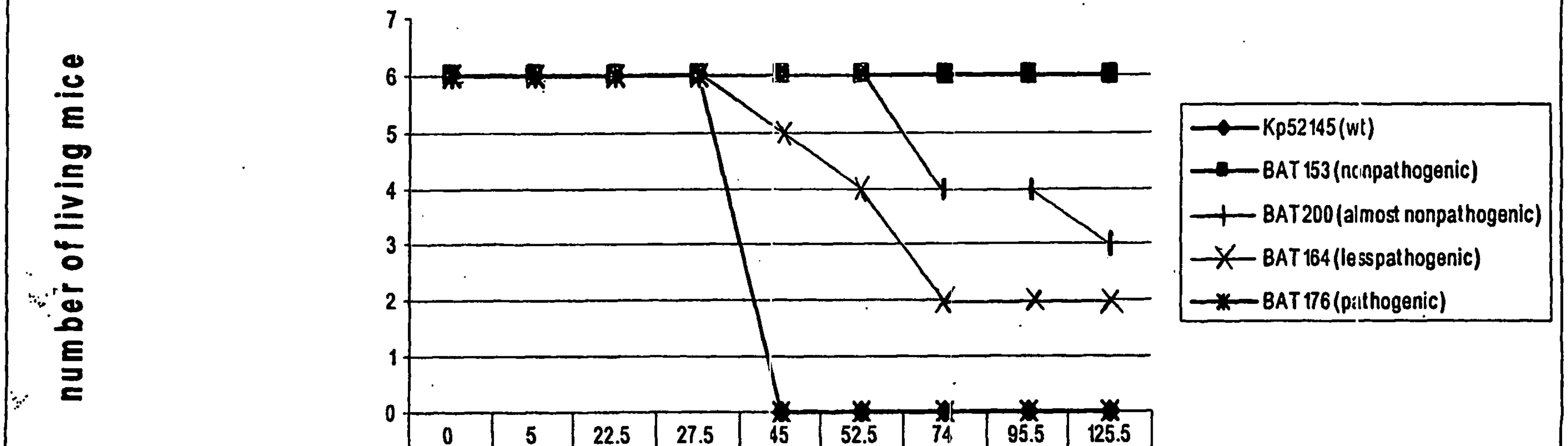


| | 0 | 5 | 22.5 | 27.5 | 45 | 52.5 | 74 | 95.5 | 125.5 |
|---|---|---|---|---|---|---|---|---|---|
| Kp52145 (wt) | 6 | 6 | 6 | 6 | 0 | 0 | 0 | 0 | 0 |
| BAT 153 (nonpathogenic) | 6 | 6 | 6 | 6 | 6 | 6 | 6 | 6 | 6 |
| BAT 200 (almost nonpathogenic) | 6 | 6 | 6 | 6 | 6 | 6 | 4 | 4 | 3 |
| BAT 164 (lesspathogenic) | 6 | 6 | 6 | 6 | 5 | 4 | 2 | 2 | 2 |
| BAT 176 (pathogenic) | 6 | 6 | 6 | 6 | 0 | 0 | 0 | 0 | 0 |

time post-infection (hour)

**Figure 13**

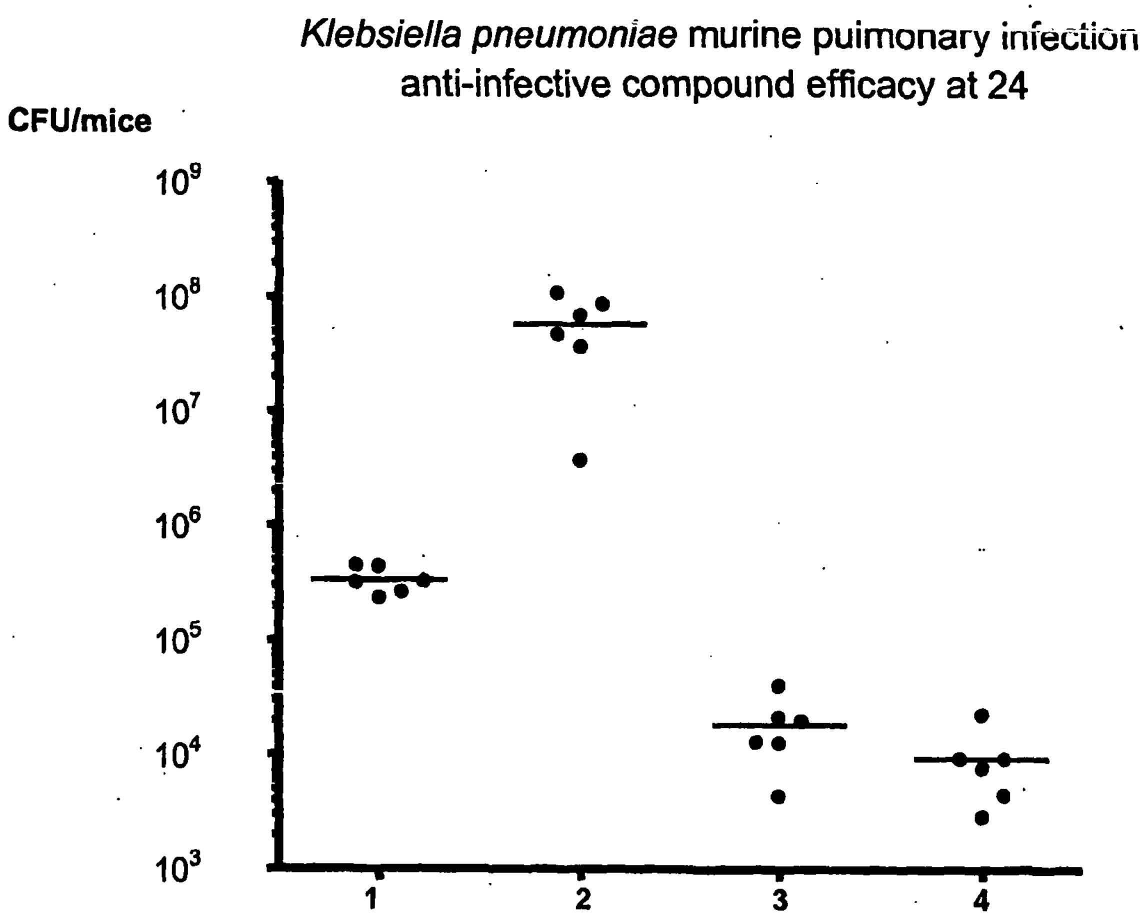
*Klebsiella pneumoniae* murine puimonary infection
anti-infective compound efficacy at 24
CFU/mice
$10^9$
$10^8$
$10^7$
$10^6$
$10^5$
$10^4$
$10^3$
1
2
3
4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9850080 A
- WO 02101081 A


### Non-patent literature cited in the description


- Bergey's Manual of Systematic Bacteriology (The Prokaryotes. 1992
- **Lavender et al.** *Int, J. Med. Micribiol.,* 2005, vol. 295 (3), 153-159
- **Zhang et al.** *J. Biomol. Screen,* 1999, vol. 4, 67-73
- **Nassif X. ; Sansonetti P.J.** *Infect Immun.,* 1986, vol. 54 (3), 603-8
- **Zhang,J. et al.** *J. Biomol. Screen.,* 1999, vol. 4, 67-73